# EUROPEAN PATENT SPECIFICATION

(11) **EP 2 416 805 B1**
(45) Date of publication and mention of the grant of the patent: **24.07.2013**
(21) Application number: 10718864.1
(22) Date of filing: 08.04.2010
(51) Int. Cl.: A61K 47/48, C07K 16/28, C07K 16/30, A61P 35/00

(54) **Amatoxin antibody conjugates for the treatment of cancer**
Amatoxin-Antikörper-Konjugate zur Behandlung von Krebs
Conjugés d'amatoxine et d'anticorps pour le traitement du cancer

(30) Priority: 08.04.2009 US 167690 P
(43) Date of publication of application: 15.02.2012
(73) Proprietor: Faulstich, Heinz, Dr., 69123 Heidelberg (DE); Deutsches Krebsforschungszentrum, 69120 Heidelberg (DE)
(72) Inventor: BREITLING, Frank, 69115 Heidelberg (DE); LÜTTGAU, Sandra, 69469 Bad Schönborn (DE); MOLDENHAUER, Gerhard, 69120 Heidelberg (DE); FAULSTICH, Heinz, Dr., 69123 Heidelberg (DE)
(74) Representative: Zwicker, Jörk
(86) International application number: PCT/EP2010/002206
(87) International publication number: WO 2010/115630

(56) References cited:
- EP-A1- 1 661 584
- EP-A1- 1 859 811
- DAVIS M T B ET AL: "A conjugate of [alpha]-amanitin and monoclonal immunoglobulin G to Thy 1.2 antigen is selectively toxic to T lymphoma cells" SCIENCE 1981 US, vol. 213, no. 4514, 1981, pages 1385-1388, XP002592035 ISSN: 0036-8075 -& PRESTON J F ET AL: "Preparation and characterization of an alpha-amanitin azo derivative with a free carboxyl group and its bovine serum albumin conjugate" ARCHIVES OF BIOCHEMISTRY AND BIOPHYSICS, ACADEMIC PRESS, US LNKD- DOI:10.1016/0003-9861(81)90257-5, vol. 209, no. 1, 1 June 1981 (1981-06-01), pages 63-71, XP024804570 ISSN: 0003-9861 [retrieved on 1981-06-01]
- ZHELEV Z ET AL: "Preparation of a beta-amanitin - concanavalin A conjugate of low toxicity" TOXICON, ELMSFORD, NY, US LNKD- DOI:10.1016/0041-0101(87)90161-9, vol. 25, no. 9, 1 January 1987 (1987-01-01), pages 981-987, XP023717796 ISSN: 0041-0101 [retrieved on 1987-01-01]
- BERMBACH U ET AL: "Epidermal growth factor labeled beta-amanitin-poly-L-ornithin: preparation and evidence for specific cytotoxicity" BIOCHEMISTRY, AMERICAN CHEMICAL SOCIETY, US LNKD- DOI:10.1021/BI00481A012, vol. 29, 1 January 1990 (1990-01-01), pages 6839-6845, XP002404130 ISSN: 0006-2960
- FAULSTICH H ET AL: "AMANITA TOXINS BOUND TO BIOPOLYMERS" PEPTIDES. PROCEEDINGS OF THE EUROPEAN PEPTIDE SYMPOSIUM, XX, XX, 1 January 1975 (1975-01-01), pages 333-338, XP008053982
- FAULSTICH H ET AL: "PROTEIN CONJUGATES OF FUNGAL TOXINS" METHODS IN ENZYMOLOGY; [METHODS IN ENZYMOLOGY], ACADEMIC PRESS INC, SAN DIEGO, CA, US LNKD- DOI:10.1016/S0076-6879(85)12019-7, vol. 112, 1 January 1985 (1985-01-01), pages 225-237, XP008053933 ISSN: 0076-6879
- WINTER M J ET AL: "The Epithelial Cell Adhesion Molecule (Ep-CAM) as a Morphoregulatory Molecule Is a Tool in Surgical Pathology" AMERICAN JOURNAL OF PATHOLOGY 200312 US, vol. 163, no. 6, December 2003 (2003-12), pages 2139-2148, XP002592036 ISSN: 0002-9440 cited in the application
- WENT P TH ET AL: "Frequent EpCam Protein Expression in Human Carcinomas" HUMAN PATHOLOGY 200401 US LNKD- DOI:10.1016/J.HUMPATH.2003.08.026, vol. 35, no. 1, January 2004 (2004-01), pages 122-128, XP002592037 ISSN: 0046-8177 cited in the application

## Description

### FIELD OF THE INVENTION

The invention relates to tumour therapy. In one aspect, the present invention relates to conjugates of target-binding moieties and toxins that are useful in the treatment of cancer. In particular, the toxin is an amatoxin, and the target-binding moieties (e.g. antibodies) are directed against tumour-associated antigens, such as epithelial cell adhesion molecule (EpCAM). In a further aspect the invention relates to pharmaceutical compositions comprising such target-binding moiety toxin conjugates and to the use of such target-binding moiety toxin conjugates for the preparation of such pharmaceutical compositions. The target-binding moiety toxin conjugates and pharmaceutical compositions of the invention are useful for the treatment of cancer, in particular adenocarcinoma, such as pancreatic cancer, cholangiocarcinoma, breast cancer, and colorectal cancer.

### BACKGROUND OF THE INVENTION AND STATE OF THE ART

### Amatoxins

Amatoxins are cyclic peptides composed of 8 amino acids. They can be isolated from *Amanita phalloides* mushrooms or prepared from the building blocks by synthesis. Amatoxins specifically inhibit the DNA-dependent RNA polymerase II of mammalian cells, and thereby also the transcription and protein biosynthesis of the affected cells. Inhibition of transcription in a cell causes stop of growth and proliferation. Though not covalently bound, the complex between amanitin and RNA-polymerase II is very tight (K_{D} = 3nM). Dissociation of amanitin from the enzyme is a very slow process what makes recovery of an affected cell unlikely. When the inhibition of transcription lasts too long, the cell will undergo programmed cell death (apoptosis).

### Epithelial cell adhesion molecule

Epithelial cell adhesion molecule (EpCAM, CD326) is one of the best-studied target antigens on human tumors (Trzpis et al., 2007; Baeuerle and Gires, 2007). It represents a type I membrane glycoprotein of 314 amino acids with an apparent molecular weight of 40 kDa (Balzar et al., 1999). It is overexpressed in the majority of adenocarcinomas (Winter et al., 2003; Went et al., 2004). In particular, EpCAM expression is enhanced in node-positive breast cancer, epithelial ovarian cancer, cholangiocarcinoma, pancreatic adenocarcinoma and squamous cell head and neck cancer. Increased EpCAM expression is indicative for a poor prognosis in breast and gallbladder carcinomas (Gastl et al., 2000; Varga et al., 2004; Spizzo et al., 2002; Spizzo et al., 2004). Importantly, EpCAM is expressed by tumor initiating or cancer stem cells in mammary, colorectal and pancreatic carcinomas (Al-Hajj et al., 2003; Dalerba et al., 2007; Li et al., 2007).

EpCAM-specific monoclonal antibodies have been used as a diagnostic tool for the detection of rare circulating tumor cells in cancer patients (Allard et al., 2004; Nagrath et al., 2007). A couple of engineered anti-EpCAM antibodies are currently investigated in clinical studies.

### Conjugates of amatoxins and antibodies

Earlier patent application EP 1 859 811 A1 (published November 28, 2007) by the inventors describes conjugates, in which β-amanitin is coupled to albumin or to the monoclonal antibodies HEA125, OKT3, and PA-1. Furthermore, the inhibitory effect of these conjugates on the proliferation of breast cancer cells (MCF-7), Burkitt's lymphoma cells (Raji), and T-lymphoma cells (Jurkat) was studied.

### TECHNICAL PROBLEMS UNDERLYING THE PRESENT INVENTION

There was a need in the prior art for target-binding moiety toxin conjugates that exert their toxic effects to target cells or tissues at much lower concentration. Furthermore, a need remained in the prior art for the treatment of other types of diseases, in particular for the treatment of other types of cancer, particularly those being therapy resistant, or poorly responding to actual tumour therapies.

The present invention fulfils these and other needs. For example, the inventors found out in the experiments underlying the present invention that conjugates comprising amatoxins and the new chimeric antibody huHEA125 are capable of inhibiting tumour cell proliferation at much lower concentrations than the conjugates described in the prior art. In particular, conjugates comprising amatoxins and the chimeric antibody huHEA125 exert their inhibitory effect at a concentration that is about one hundredth of the concentration needed when using conjugates of the prior art. Furthermore, the inventors discovered that conjugates comprising amatoxins and EpCAM-specific antibodies cannot only inhibit proliferation of breast cancer cells but are surprisingly also capable of inhibiting proliferation of pancreatic adenocarcinoma cells, colorectal cancer cells, and cholangiocarcinoma cells. Additionally, the inventors found out that choosing a particular linkage point in the amatoxin part of the conjugates yields highly effective target-binding moiety toxin conjugates (in particular antibody toxin conjugates) that exert their toxic activity on the target cells at very low concentrations (IC₅₀ around 2x10⁻¹² to 2x10⁻¹¹ M) and that are highly specific for their target cells. Without wishing to be bound by a particular theory, this latter advantage might be explained in that the amatoxin is efficiently released from the target-binding moiety amatoxin conjugate inside the target cell but not outside the cell.

The above overview does not necessarily describe all problems solved by the present invention.

### SUMMARY OF THE INVENTION

In a first aspect the present invention relates to an antibody toxin conjugate for the treatment of pancreatic cancer, cholangiocarcinoma, or colorectal cancer in a patient, wherein the conjugate comprises (i) an antibody or antigen binding fragment thereof specifically binding to an epitope of epithelial cell adhesion molecule (EpCAM); (ii) an amatoxin; and (iii) optionally a linker L1.

In a second aspect the present invention relates to an antibody toxin conjugate comprising (i) an antibody or an antigen binding fragment thereof specifically binding to epithelial cell adhesion molecule (EpCAM), wherein the antibody or an antigen binding fragment thereof comprises: (a) the heavy chain of huHEA125, wherein the heavy chain is selected from the group consisting of: (a1) the membrane-bound form of the heavy chain according to SEQ ID NO: 1, wherein the variable domain of the heavy chain VH as shown in SEQ ID NO: 3 comprises between 0 and 10 amino acid exchanges, between 0 and 10 amino acid deletions and/or between 0 and 10 amino acid additions positioned in the framework regions of VH, and wherein the constant domain of the heavy chain as shown in SEQ ID NO: 26 comprises between 0 and 10 amino acid exchanges, between 0 and 10 amino acid deletions and/or between 0 and 10 amino acid additions; and (a2) the soluble form of the heavy chain according to SEQ ID NO: 2, wherein the variable domain of the heavy chain VH as shown in SEQ ID NO: 3 comprises between 0 and 10 amino acid exchanges, between 0 and 10 amino acid deletions and/or between 0 and 10 amino acid additions positioned in the framework regions of VH, and wherein the constant domain of the heavy chain as shown in SEQ ID NO: 27 comprises between 0 and 10 amino acid exchanges, between 0 and 10 amino acid deletions and/or between 0 and 10 amino acid additions; and (b) the light chain of huHEA125 according to SEQ ID NO: 11, wherein the variable domain of the light chain VL as shown in SEQ ID NO: 12 comprises between 0 and 10 amino acid exchanges, between 0 and 10 amino acid deletions and/or between 0 and 10 amino acid additions positioned in the framework regions of VL, and wherein the constant domain of the light chain CL as shown in SEQ ID NO: 28 comprises between 0 and 10 amino acid exchanges, between 0 and 10 amino acid deletions and/or between 0 and 10 amino acid additions; (ii) an amatoxin; and (iii) optionally a linker L2.

In a third aspect the present invention relates to an antibody toxin conjugate according to the second aspect for use in medicine.

In a fourth aspect the present invention relates to an antibody toxin conjugate according to the second aspect for the treatment of cancer in a patient, wherein the cancer is selected from the group consisting of pancreatic cancer, cholangiocarcinoma, breast cancer and colon cancer.

In a fifth aspect the present invention relates to a target-binding moiety toxin conjugate comprising: (i) a target-binding moiety; (ii) an amatoxin; and (iii) optionally a linker L3; wherein the amatoxin is connected to the target-binding moiety or, if present, to the linker L3 via the δ C-atom of amatoxin amino acid 3.

In an sixth aspect the present invention relates to a target-binding moiety toxin conjugate according to the fifth aspect for use in medicine.

In a seventh aspect the present invention relates to a target-binding moiety toxin conjugate according to the fifth aspect for the treatment of cancer in a patient, wherein the cancer is selected from the group consisting of pancreatic cancer, cholangiocarcinoma, breast cancer, colorectal cancer, lung cancer, prostate cancer, ovarian cancer, stomach cancer, kidney cancer, malignant melanoma, leukemia and malignant lymphoma.

In an eighth aspect the present invention relates to a pharmaceutical composition comprising the antibody toxin conjugate according to the first aspect or the second aspect or the target-binding moiety toxin conjugate according to the fifth aspect and further comprising one or more pharmaceutically acceptable diluents, carriers, excipients, fillers, binders, lubricants, glidants, disintegrants, adsorbents; and/or preservatives.

This summary of the invention does not necessarily describe all features of the invention.

### BRIEF DESCRIPTION OF THE DRAWINGS

**Fig. 1** shows the structural formulae of different amatoxins. The numbers in bold type (1 to 8) designate the standard numbering of the eight amino acids forming the amatoxin. The most important carbon atoms in amino acid 3 are labelled with Greek letter α, β, γ, and δ. The atom numbers in the side chain of the (substituted) tryptophan, i.e. amino acid no. 4, are also shown (numbers 1' to 7').
**Fig. 2** shows a comparison of the binding affinities of huHEA125-Ama and huHEA125 to target cells by a binding competition analysis. EpCAM-expressing Colo205 cells were incubated with a fixed amount of directly FITC-labeled mouse HEA125 antibody. Binding to target cells was analyzed by flow cytometry. Competition of binding with increasing amounts of huHEA125-Ama or huHEA125 revealed a very similar affinity towards the target antigen.

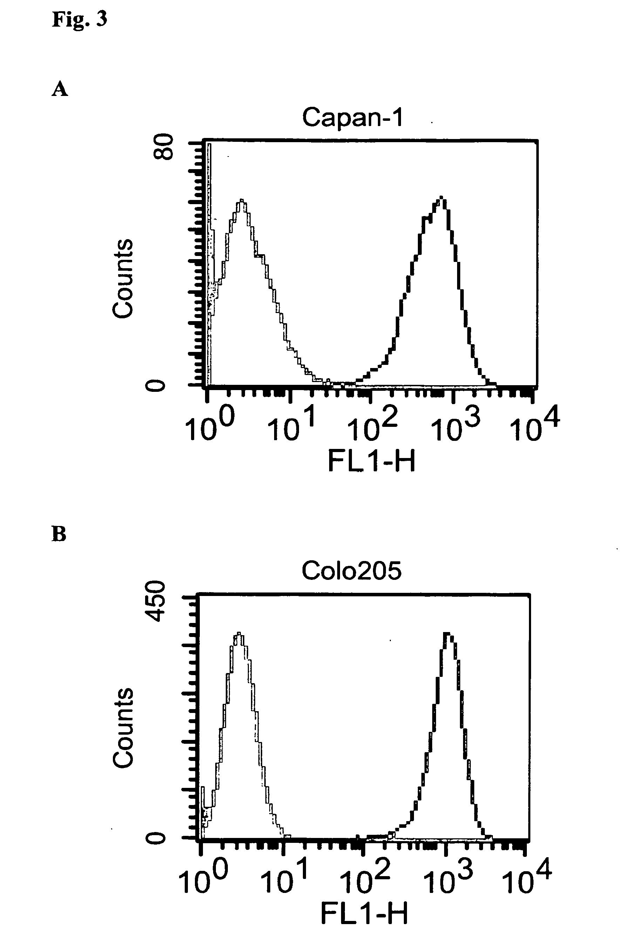

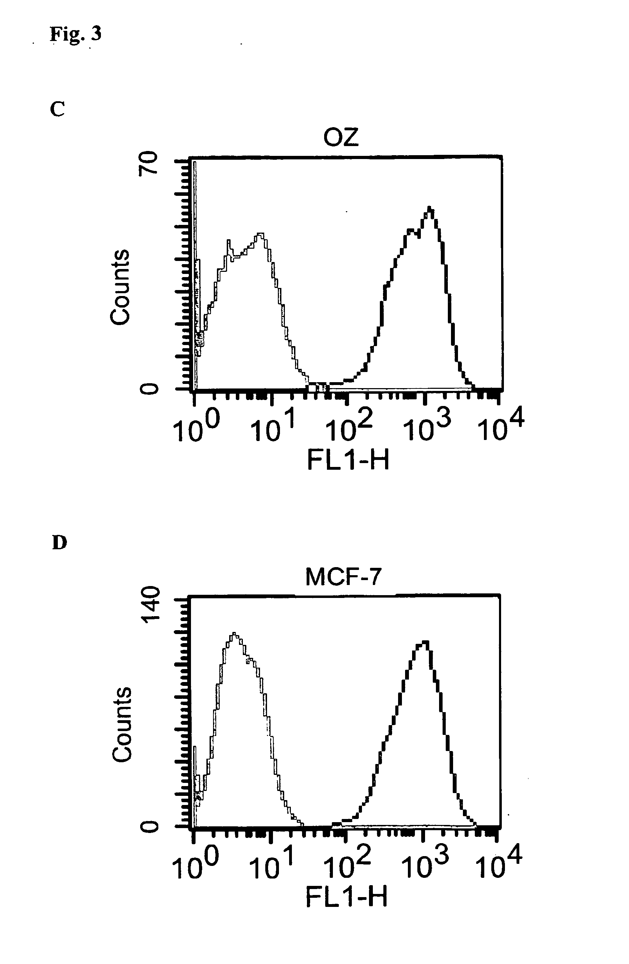

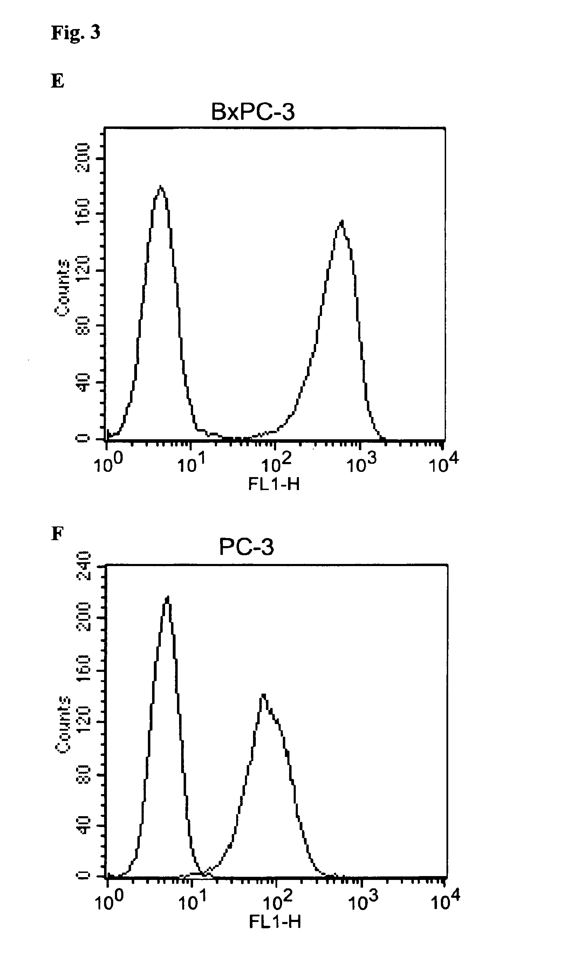

**Fig. 3** shows the surface expression of EpCAM antigen on various carcinoma cell lines detected by indirect immunofluorescence: Fig. 3A Capan-1 (human pancreatic adenocarcinoma); Fig.3B Colo205 (human colon adenocarcinoma); Fig.3C OZ (human cholangiocarcinoma); and Fig. 3D MCF-7 (human breast adenocarcinoma line), Fig. 3E BxPC-3 (human pancreatic adenocarcinoma); and Fig. 3F PC-3 (human prostate adenocarcinoma). The grey-shaded histograms on the left side of each diagram show the results obtained with control antibody Xolair®; the histograms having a white area on the right side of each diagram show the results obtained with antibody huHEA125.
**Fig. 4** shows a comparison of the inhibition of Capan-1 cell proliferation caused by Amanitin-armed antibody huHEA125, Amanitin-armed control antibody Xolair®, and free Amanitin.
**Fig. 5** shows a comparison of the inhibition of Colo205 cell proliferation caused by Amanitin-armed antibody huHEA125, Amanitin-armed control antibody Xolair®, and free Amanitin.
**Fig. 6** shows a comparison of the inhibition of MCF-7 cell proliferation caused by Amanitin-armed antibody huHEA125, Amanitin-armed control antibody Xolair®, and free Amanitin.
**Fig. 7** shows a comparison of the inhibition of OZ cell proliferation caused by Amanitin-armed antibody huHEA125, Amanitin-armed control antibody Xolair®, and free Amanitin.
**Fig. 8** shows the inhibition of BxPC-3 cell proliferation caused by Amanitin-armed antibody huHEA125, and Amanitin-armed control antibody Xolair® and free Amanitin for comparison.
**Fig. 9** shows growth inhibition of BxPC-3 tumor xenografts in NOD/SCID mice after huHEA125-amanitin treatment.
**Fig. 10** shows growth inhibition of PC-3 tumor xenografts in NOD/SCID mice after huHEA125-amanitin treatment.

### DETAILED DESCRIPTION OF THE INVENTION

### Definitions

Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification.

The term "target-binding moiety", as used herein, refers to any molecule or part of a molecule that can specifically bind to a target molecule or target epitope. Preferred target-binding moieties in the context of the present application are (i) antibodies or antigen-binding fragments thereof; (ii) antibody-like proteins; and (iii) nucleic acid aptamers. "Target-binding moieties" suitable for use in the present invention typically have a molecular mass of 40 000 Da (40 kDa) or more.

In the context of the present application the terms "target molecule" and "target epitope", respectively, refers to an antigen and an epitope of an antigen, respectively, that is specifically bound by a target-binding moiety, preferably the target molecule is a tumour-associated antigen, in particular an antigen or an epitope, which is present on the surface of one or more tumour cell types in an increased concentration and/or in a different steric configuration as compared to the surface of non-tumour cells. Preferably, said antigen or epitope is present on the surface of one or more tumour cell types but not on the surface of non-tumour cells.

The term "antibody or antigen binding fragment thereof", as used herein, refers to immunoglobulin molecules and immunologically active portions of immunoglobulin molecules, i.e. molecules that contain an antigen binding site that immunospecifically binds an antigen. Also comprised are immunoglobulin-like proteins that are selected through techniques including, for example, phage display to specifically bind to a target molecule, e.g. to the target protein EpCAM. The immunoglobulin molecules of the invention can be of any type (e.g., IgG, IgE, IgM, IgD, IgA and IgY), class (e.g., IgG1, IgG2, IgG3, IgG4, IgA1 and IgA2) or subclass of immunoglobulin molecule. "Antibodies and antigen-binding fragments thereof" suitable for use in the present invention include polyclonal, monoclonal, monovalent, bispecific, heteroconjugate, multispecific, human, humanized (in particular CDR-grafted), deimmunized, or chimeric antibodies, single chain antibodies (e.g. scFv), Fab fragments, F(ab')₂ fragments, fragments produced by a Fab expression library, diabodies or tetrabodies (Holliger P. et al., 1993), nanobodies, anti-idiotypic (anti-Id) antibodies (including, e.g., anti-Id antibodies to antibodies of the invention), and epitope-binding fragments of any of the above.

In some embodiments the antigen-binding fragments are human antigen-binding antibody fragments of the present invention and include Fab, Fab' and F(ab')₂, Fd, single-chain Fvs (scFv), single-chain antibodies, disulfide-linked Fvs (dsFv) and fragments comprising either a VL or VH domain. Antigen-binding antibody fragments, including single-chain antibodies, may comprise the variable domain(s) alone or in combination with the entirety or a portion of the following: hinge region, CL, CH1, CH2, and CH3 domains. Also included in the invention are antigen-binding fragments also comprising any combination of variable domain(s) with a hinge region, CL, CH1, CH2, and CH3 domains.

Antibodies usable in the invention may be from any animal origin including birds and mammals. Preferably, the antibodies are human, rodent (e.g. mouse and rat), donkey, sheep rabbit, goat, guinea pig, camel, horse, or chicken. It is particularly preferred that the antibodies are of human or murine origin. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins, as described for example in U.S. Patent No. 5,939,598 by Kucherlapati & Jakobovits.

The term "antibody-like protein" refers to a protein that has been engineered (e.g. by mutagenesis of loops) to specifically bind to a target molecule. Typically, such an antibody-like protein comprises at least one variable peptide loop attached at both ends to a protein scaffold. This double structural constraint greatly increases the binding affinity of the antibody-like protein to levels comparable to that of an antibody. The length of the variable peptide loop typically consists of 10 to 20 amino acids. The scaffold protein may be any protein having good solubility properties. Preferably, the scaffold protein is a small globular protein. Antibody-like proteins include affibodies, anticalins, and designed ankyrin repeat proteins (for review see: Binz et al. 2005). Antibody-like proteins can be derived from large libraries of mutants, e.g. be panned from large phage display libraries and can be isolated in analogy to regular antibodies. Also, antibody-like binding proteins can be obtained by combinatorial mutagenesis of surface-exposed residues in globular proteins.

The term "nucleic acid aptamer" refers to a nucleic acid molecule that has been engineered through repeated rounds of *in vitro selection* or SELEX (systematic evolution of ligands by exponential enrichment) to bind to a target molecule (for a review see: Brody and Gold, 2000). The nucleic acid aptamer may be a DNA or RNA molecule. The aptamers may contain modifications, e.g. modified nucleotides such as 2'-fluorine-substituted pyrimidines.

The term "amatoxin" includes all cyclic peptides composed of 8 amino acids as isolated from the genus *Amanita* and described in ref. (Wieland, T. and Faulstich H., 1978); further all chemical derivatives thereof; further all semisynthetic analogs thereof; further all synthetic analogs thereof built from building blocks according to the master structure of the natural compounds (cyclic, 8 aminoacids), further all synthetic or semisynthetic analogs containing non-hydroxylated amino acids instead of the hydroxylated amino acids, further all synthetic or semisynthetic analogs, in which the thioether sulfoxide moiety is replaced by a sulfide, sulfone, or by atoms different from sulfur, e.g. a carbon atom as in a carbaanalog of amanitin.

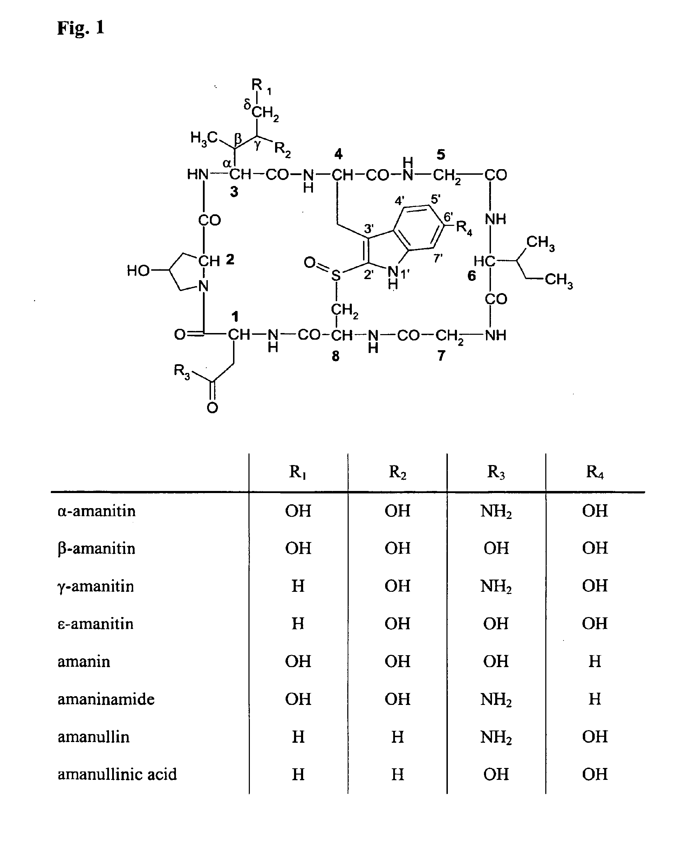

Functionally, amatoxins are defined as peptides or depsipeptides that inhibit mammalian RNA polymerase II. Preferred amatoxins are those with a functional group (e.g. a carboxylic group, an amino group, a hydroxy group, a thiol or a thiol-capturing group) that can be reacted with linker molecules or proteins, such as antibodies or antibody fragments. Amatoxins which are particularly suitable for the conjugates of the present invention are α-amanitin, β-amanitin, γ-amanitin, ε-amanitin, amanin, amaninamide, amanullin, and amanullinic acid as shown in Fig. 1 as well as salts, chemical derivatives, semisynthetic analogs, and synthetic analogs thereof. Particularly preferred amatoxins for use in the present invention are α-amanitin, β-amanitin, and amaninamide.

As used herein, a "derivative" of a compound refers to a species having a chemical structure that is similar to the compound, yet containing at least one chemical group not present in the compound and/or deficient of at least one chemical group that is present in the compound. The compound to which the derivative is compared is known as the "parent" compound. Typically, a "derivative" may be produced from the parent compound in one or more chemical reaction steps.

As used herein, an "analog" of a compound is structurally related but not identical to the compound and exhibits at least one activity of the compound. The compound to which the analog is compared is known as the "parent" compound. The afore-mentioned activities include: binding activity to another compound; inhibitory activity, e.g. enzyme inhibitory activity; toxic effects; activating activity, e.g. enzyme-activating activity. It is not required that the analog exhibits such an activity to the same extent as the parent compound. A compound is regarded as an analog within the context of the present application, if it exhibits the relevant activity to a degree of at least 1% (more preferably at least 5%, more preferably at least 10%, more preferably at least 20%, more preferably at least 30%, more preferably at least 40%, and more preferably at least 50%) of the activity of the parent compound. Thus, an "analog of an amatoxin", as it is used herein, refers to a compound that is structurally related to any one of α-amanitin, β-amanitin, γ-amanitin, ε-amanitin, amanin, amaninamide, amanullin, and amanullinic acid as shown in Fig. 1 and that exhibits at least 1% (more preferably at least 5%, more preferably at least 10%, more preferably at least 20%, more preferably at least 30%, more preferably at least 40%, and more preferably at least 50%) of the inhibitory activity against mammalian RNA polymerase II as compared to at least one of α-amanitin, β-amanitin, γ-amanitin, ε-amanitin, amanin, amaninamide, amanullin, and amanullinic acid. An "analog of an amatoxin" suitable for use in the present invention may even exhibit a greater inhibitory activity against mammalian RNA polymerase II than any one of α-amanitin, β-amanitin, γ-amanitin, ε-amanitin, amanin, amaninamide, amanullin, or amanullinic acid. The inhibitory activity might be measured by determining the concentration at which 50% inhibition occurs (IC₅₀ value).

A "linker" in the context of the present application refers to a molecule that increases the distance between two components, e.g. to alleviate steric interference between the target-binding moiety and the amatoxin, which may otherwise decrease the ability of the amatoxin to interact with RNA polymerase II. The linker may serve another purpose as it may facilitate the release of the amatoxin specifically in the cell being targeted by the target binding moiety. It is preferred that the linker and preferably the bond between the linker and the amatoxin on one side and the bond between the linker and the antibody on the other side is stable under the physiological conditions outside the cell, e.g. the blood, while it can be cleaved inside the cell, in particular inside the target cell, e.g. cancer cell or immune cell. To provide this selective stability the linker may comprise functionalities that are preferably pH-sensitive to generate pH-sensitive linkers as described, e.g. in S. Fletcher, M. R. Jorgensens and A. D. Miller; Org. Lett. 2004, 6(23), pp 4245-4248, or protease sensitive to generate protease sensitive linkers as described, e.g. in L. DA Ibsen, Blood 2003, 102, 1458-65 or Francisco JA, Cerreny CG, Meyer DL, Nat. Biotechnol 2003, 21, 778-84. Alternatively, the bond linking the linker to the target binding moiety may provide the selective stability. Preferably a linker has a length of at least 1, preferably of 1-20 atoms length (e.g. 1, 2, 3, 4, 5, 6, 7, 8, 9, 10, 11, 12, 13, 14, 15, 16, 17, 18, 19, or 20 atoms) wherein one side of the linker has been reacted with the amatoxin and, the other side with a target-binding moiety. In the context of the present invention, a linker preferably is a C₁₋₂₀-alkyl, C₁₋₂₀-heteroalkyl, C₂₋₂₀-alkenyl, C₂₋₂₀-heteroalkenyl, C₂₋₂₀-alkynyl, C₂₋₂₀-heteroalkynyl, cycloalkyl, heterocycloalkyl, aryl, heteroaryl, aralkyl, or a heteroaralkyl group, optionally substituted. The linker may contain one or more structural elements such as amide, ester, ether, thioether, disulfide, or hydrocarbon moieties. The linker may also contain combinations of two or more of these structural elements. Each one of these structural elements may be present in the linker more than once, e.g. twice, three times, four times, five times, or six times. In some embodiments the linker may comprise a disulfide bond. It is understood that the linker has to be attached either in a single step or in two or more subsequent steps to the amatoxin and the target binding moiety. To that end the linker to be will carry two groups, preferably at a proximal and distal end, which can (i) form a covalent bond to a group, preferably an activated group on an amatoxin or the target binding-peptide or (ii) which is or can be activated to form a covalent bond with a group on an amatoxin. Accordingly, if the linker is present, it is preferred that chemical groups are at the distal and proximal end of the linker, which are the result of such a coupling reaction, e.g. an ester, an ether, a urethane, or a peptide bond. The presence of a "linker" is optional, i.e. the toxin may be directly linked to a residue of the target-binding moiety in some embodiments of the target-binding moiety toxin conjugate of the present invention. It is preferred that the linker is connected directly via a bond to the targeting moiety, preferably at its terminus. If the target-binding moiety comprises free amino, carboxy or sulfhydryl groups, e.g. in the form of Asp, Glu, Arg, Lys, Cys residues, which may be comprised in a polypeptide, than it is preferred that the linker is coupled to such a group.

As used herein, a first compound (e.g. an antibody) is considered to "specifically bind" to a second compound (e.g. an antigen, such as a target protein), if it has a dissociation constant K_{D} to said second compound of 100 µM or less, preferably 50 µM or less, preferably 30 µM or less, preferably 20 µM or less, preferably 10 µM or less, preferably 5 µM or less, more preferably 1 µM or less, more preferably 900 nM or less, more preferably 800 nM or less, more preferably 700 nM or less, more preferably 600 nM or less, more preferably 500 nM or less, more preferably 400 nM or less, more preferably 300 nM or less, more preferably 200 nM or less, even more preferably 100 nM or less, even more preferably 90 nM or less, even more preferably 80 nM or less, even more preferably 70 nM or less, even more preferably 60 nM or less, even more preferably 50 nM or less, even more preferably 40 nM or less, even more preferably 30 nM or less, even more preferably 20 nM or less, and even more preferably 10 nM or less.

As used herein, a "patient" means any mammal or bird who may benefit from a treatment with the target-binding moiety toxin conjugates described herein. Preferably, a "patient" is selected from the group consisting of laboratory animals (e.g. mouse or rat), domestic animals (including e.g. guinea pig, rabbit, donkey, sheep, goat, chicken, camel, horse, cat, or dog), or primates including human beings. It is particularly preferred that the "patient" is a human being.

As used herein, "treat", "treating" or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "administering" includes *in vivo* administration, as well as administration directly to tissue *ex vivo,* such as vein grafts.

An "effective amount" is an amount of a therapeutic agent sufficient to achieve the intended purpose. The effective amount of a given therapeutic agent will vary with factors such as the nature of the agent, the route of administration, the size and species of the animal to receive the therapeutic agent, and the purpose of the administration. The effective amount in each individual case may be determined empirically by a skilled artisan according to established methods in the art.

"Pharmaceutically acceptable" means approved by a regulatory agency of the Federal or a state government or listed in the U.S. Pharmacopeia or other generally recognized pharmacopeia for use in animals, and more particularly in humans.

### Embodiments of the Invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect the present invention is directed to an antibody toxin conjugate for the treatment of pancreatic cancer, cholangiocarcinoma, or colorectal cancer in a patient, wherein the conjugate comprises (i) an antibody or antigen binding fragment thereof specifically binding to an epitope of epithelial cell adhesion molecule (EpCAM); (ii) an amatoxin; and (iii) optionally a linker.

In a preferred embodiment of the first aspect the antibody or antigen binding fragment thereof is selected from a diabody, a tetrabody, a nanobody, a chimeric antibody, a deimmunized antibody, a humanized antibody or a human antibody. In a preferred embodiment of the first aspect the antigen binding fragment is selected from the group consisting of Fab, F(ab')₂, Fd, Fv, single-chain Fv, and disulfide-linked Fvs (dsFv).

In a preferred embodiment the epitope of EpCAM is an epitope of human EpCAM.

In a preferred embodiment of the first aspect the antibody or the antigen binding fragment thereof comprises (a) the CDR3 domain (SEQ ID NO: 22) of the heavy chain of huHEA125; and/or (b) the CDR3 domain (SEQ ID NO: 25) of the light chain of huHEA125. In a particularly preferred embodiment, the antibody or the antigen binding fragment thereof comprises both of these CDR3 domains as set forth in SEQ ID NO: 22 and SEQ ID NO: 25. Preferably, the antibody or the antigen binding fragment thereof additionally comprises one or more of the following: (a) the CDR2 domain (SEQ ID NO: 21) of the heavy chain of huHEA125; (b) the CDR1 domain (SEQ ID NO: 20) of the heavy chain of huHEA125; (c) the CDR2 domain (SEQ ID NO: 24) of the light chain of huHEA125; and (d) the CDR1 domain (SEQ ID NO: 23) of the light chain of huHEA125. In a preferred embodiment the antibody or the antigen binding fragment thereof comprises the CDR3 domain (SEQ ID NO: 22), the CDR2 domain (SEQ ID NO: 21), and the CDR1 domain (SEQ ID NO: 20) of the heavy chain of huHEA125. In a preferred embodiment the antibody or the antigen binding fragment thereof comprises the CDR3 domain (SEQ ID NO: 25), the CDR2 domain (SEQ ID NO: 24), and the CDR1 domain (SEQ ID NO: 23) of the light chain of huHEA125. In a particularly preferred embodiment, the antibody or the antigen binding fragment thereof comprises the CDR3 domains, the CDR2 domains, and the CDR1 domains of the heavy chain and the light chain, i.e. the antibody or the antigen binding fragment thereof comprises the amino acid sequences as set forth in SEQ ID NO: 20, 21, 22, 23, 24, and 25.

In a preferred embodiment of the first aspect the antibody or the antigen binding fragment thereof comprises the variable domain of the heavy chain (= VH) of huHEA125 (SEQ ID NO: 3) and/or variable domain of the light chain (= VL) of huHEA125 (SEQ ID NO: 12). In a particularly preferred embodiment, the antibody or the antigen binding fragment thereof comprises both the VH domain (SEQ ID NO: 3) and the VL domain (SEQ ID NO: 12) of huHEA125.

In a preferred embodiment of the first aspect the antibody or the antigen binding fragment thereof comprises the heavy chain of huHEA125 (soluble form, SEQ ID NO: 2) and/or the light chain of huHEA125 (SEQ ID NO: 11). In one embodiment, the heavy chain of huHEA125 and/or the light chain of huHEA125 each comprise independently from each other up to 20 (e.g. 1, 2, 3, 4, 5, 6, 7, 8, 9, 10, 11, 12, 13, 14, 15, 16, 17, 18, 19 or 20) amino acid exchanges, deletions, or additions, wherein these amino acid exchanges, deletions, or additions may be positioned in the constant domains of the heavy chain and/or in the constant domain of the light chain and/or in the framework regions of the variable domain of the heavy chain and/or in the framework regions of the variable domain of the light chain. In a particularly preferred embodiment, the antibody is a complete IgG antibody comprising two heavy chains of huHEA125 (SEQ ID NO: 2) and two light chains of huHEA125 (SEQ ID NO: 11), wherein one heavy chain is connected to one light chain via a disulfide linkage and wherein the heavy chains are connected to each other by one or two (preferably two) disulfide linkages.

In a preferred embodiment of the first aspect the antibody or the antigen binding fragment thereof comprises the heavy chain of huHEA125 (membrane-bound form, SEQ ID NO: 1) and/or the light chain of huHEA125 (SEQ ID NO: 11). In one embodiment, the heavy chain of huHEA125 and/or the light chain of huHEA125 each comprise independently from each other up to 20 (e.g. 1, 2, 3, 4, 5, 6, 7, 8, 9, 10, 11, 12, 13, 14, 15, 16, 17, 18, 19 or 20) amino acid exchanges, deletions, or additions, wherein these amino acid exchanges, deletions, or additions may be positioned in the constant domains of the heavy chain and/or in the constant domain of the light chain and/or in the framework regions of the variable domain of the heavy chain and/or in the framework regions of the variable domain of the light chain. In a particularly preferred embodiment, the antibody is a complete IgG antibody comprising two heavy chains of huHEA125 (SEQ ID NO: 1) and two light chains of huHEA125 (SEQ ID NO: 11), wherein one heavy chain is connected to one light chain via a disulfide linkage and wherein the heavy chains are connected to each other by one or two (preferably two) disulfide linkages.

In a preferred embodiment of the first aspect the amatoxin is selected from α-amanitin, β-amanitin, γ-amanitin, ε-amanitin, amanin, amaninamide, amanullin, and amanullinic acid (all shown in Fig. 1), as well as salts, chemical derivatives, semisynthetic analogs, and synthetic analogs thereof. Particularly preferred amatoxins are α-amanitin, β-amanitin, and amaninamide, as well as salts, chemical derivatives, semisynthetic analogs, and synthetic analogs thereof.

In a preferred embodiment of the first aspect the amatoxin is connected to the antibody or, if present, to the linker L1 via the δC-atom of amatoxin amino acid 3 (see Fig. 1). In preferred amatoxins usable in the present invention said amino acid 3 is isoleucine, γ-hydroxy-isoleucine or γ,δ-dihydroxy-isoleucine.

In preferred embodiments of the first aspect, the amatoxin is connected to the antibody or, if present, to the linker L1 via an oxygen atom bound to the δC-atom of amatoxin amino acid 3. It is further preferred that the amatoxin is connected to the antibody or, if present, to the linker L1 via an ester linkage, an ether linkage or a urethane linkage. In these embodiments, it is preferred that amino acid 3 is γ,δ-dihydroxy-isoleucine.

In preferred embodiments of the first aspect, the antibody is connected to the amatoxin or, if present, to the linker L1 via an amino group present in the antibody.

In a preferred embodiment of the first aspect the linker L1 is an alkyl, heteroalkyl, alkenyl, heteroalkenyl, alkynyl, heteroalkynyl, cycloalkyl, heterocycloalkyl, aryl, heteroaryl, aralkyl, or a heteroaralkyl group, optionally substituted. In further preferred embodiments of the first aspect the linker L1 comprises a disulfide bond.

In a second aspect the present invention is directed to an antibody toxin conjugate comprising (i) an antibody or an antigen binding fragment thereof specifically binding to epithelial cell adhesion molecule (EpCAM), wherein the antibody or an antigen binding fragment thereof comprises: (a) the heavy chain of huHEA125, wherein the heavy chain is selected from the group consisting of: (a1) the membrane-bound form of the heavy chain according to SEQ ID NO: 1, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions positioned in the framework regions of VH, and wherein the constant domain of the heavy chain as shown in SEQ ID NO: 26 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions; and (a2) the soluble form of the heavy chain according to SEQ ID NO: 2, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1,2, 3, 4, 5, 6, 7, 8, 9, or 10) amino acid additions positioned in the framework regions of VH, and wherein the constant domain of the heavy chain as shown in SEQ ID NO: 27 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions; and (b) the light chain of huHEA125 according to SEQ ID NO: 11, wherein the variable domain of the light chain VL as shown in SEQ ID NO: 12 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions positioned in the framework regions of VL, and wherein the constant domain of the light chain CL as shown in SEQ ID NO: 28 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions; (ii) an amatoxin; and (iii) optionally a linker.

In a preferred embodiment of the second aspect the antibody or an antigen binding fragment thereof comprises: (a) the heavy chain of huHEA125, wherein the heavy chain is selected from the group consisting of: (a1) the membrane-bound form of the heavy chain according to SEQ ID NO: 1, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions positioned in the framework regions of VH; and (a2) the soluble form of the heavy chain according to SEQ ID NO: 2, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions positioned in the framework regions of VH; and (b) the light chain of huHEA125 according to SEQ ID NO: 11, wherein the variable domain of the light chain VL as shown in SEQ ID NO: 12 comprises between 0 and 10 (e.g. 0, 1, 2, 3, 4, 5, 6, 7, 8, 9, or 10) amino acid exchanges, between 0 and 10 (e.g. 0, 1, 2, 3, 4, 5, 6, 7, 8, 9, or 10) amino acid deletions and/or between 0 and 10 (e.g. 0, 1, 2, 3, 4, 5, 6, 7, 8, 9, or 10) amino acid additions positioned in the framework regions of VL.

In a preferred embodiment of the second aspect the antibody or an antigen binding fragment thereof comprises: (a) the heavy chain of huHEA125, wherein the heavy chain is selected from the group consisting of: (a1) the membrane-bound form of the heavy chain according to SEQ ID NO: 1, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges, amino acid deletions and/or amino acid additions positioned in the framework regions of VH, and wherein the constant domain of the heavy chain as shown in SEQ ID NO: 26 comprises between 0 and 10 (e.g. 0, 1, 2, 3, 4, 5, 6, 7, 8, 9, or 10) amino acid exchanges, amino acid deletions and/or amino acid additions; and (a2) the soluble form of the heavy chain according to SEQ ID NO: 2, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges, amino acid deletions and/or amino acid additions positioned in the framework regions of VH, and wherein the constant domain of the heavy chain as shown in SEQ ID NO: 27 comprises between 0 and 10 (e.g. 0, 1, 2, 3, 4, 5, 6, 7, 8, 9, or 10) amino acid exchanges, amino acid deletions and/or amino acid additions; and (b) the light chain of huHEA125 according to SEQ ID NO: 11, wherein the variable domain of the light chain VL as shown in SEQ ID NO: 12 comprises between 0 and 10 (e.g. 0, 1, 2, 3, 4, 5, 6, 7, 8, 9, or 10) amino acid exchanges, amino acid deletions and/or amino acid additions positioned in the framework regions of VL, and wherein the constant domain of the light chain CL as shown in SEQ ID NO: 28 comprises between 0 and 10 (e.g. 0, 1, 2, 3, 4, 5, 6, 7, 8, 9, or 10) amino acid exchanges, amino acid deletions and/or amino acid additions.

In a preferred embodiment of the second aspect the antibody or an antigen binding fragment thereof comprises: (a) the heavy chain of huHEA125, wherein the heavy chain is selected from the group consisting of: (a1) the membrane-bound form of the heavy chain according to SEQ ID NO: 1, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges positioned in the framework regions of VH, and wherein the constant domain of the heavy chain as shown in SEQ ID NO: 26 comprises between 0 and 10 (e.g. 0, 1, 2, 3, 4, 5, 6, 7, 8, 9, or 10) amino acid exchanges; and (a2) the soluble form of the heavy chain according to SEQ ID NO: 2, wherein the variable domain of the heavy chain VH as shown in SEQ ID NO: 3 comprises between 0 and 10 (e.g. 0, 1, 2, 3, 4, 5, 6, 7, 8, 9, or 10) amino acid exchanges positioned in the framework regions of VH, and wherein the constant domain of the heavy chain as shown in SEQ ID NO: 27 comprises between 0 and 10 (e.g. 0, 1, 2, 3, 4, 5, 6, 7, 8, 9, or 10) amino acid exchanges; and (b) the light chain of huHEA125 according to SEQ ID NO: 11, wherein the variable domain of the light chain VL as shown in SEQ ID NO: 12 comprises between 0 and 10 (e.g. 0, 1, 2, 3, 4, 5, 6, 7, 8, 9, or 10) amino acid exchanges positioned in the framework regions of VL, and wherein the constant domain of the light chain CL as shown in SEQ ID NO: 28 comprises between 0 and 10 (e.g. 0, 1, 2, 3, 4, 5, 6, 7, 8, 9, or 10) amino acid exchanges.

In a preferred embodiment of the second aspect the antibody or an antigen binding fragment thereof comprises: (a) the heavy chain of huHEA125, wherein the heavy chain is selected from the group consisting of: (a1) the membrane-bound form of the heavy chain according to SEQ ID NO: 1; and (a2) the soluble form of the heavy chain according to SEQ ID NO: 2; and (b) the light chain of huHEA125 according to SEQ ID NO: 11.

In a preferred embodiment of the second aspect the antibody or antigen binding fragment thereof is selected from a chimeric antibody, a deimmunized antibody, a humanized antibody or a human antibody. In a preferred embodiment of the second aspect the antigen binding fragment is selected from the group consisting of Fab, F(ab')₂, and Fd.

In a preferred embodiment of the second aspect the antibody is huHEA 125 or an antigen binding fragment thereof.

In a preferred embodiment of the second aspect the antibody or antigen binding fragment thereof specifically binds to human EpCAM.

In a preferred embodiment of the second aspect the amatoxin is selected from α-amanitin, β-amanitin, γ-amanitin, ε-amanitin, amanin, amaninamide, amanullin, and amanullinic acid (all shown in Fig. 1), as well as salts, chemical derivatives, semisynthetic analogs, and synthetic analogs thereof. Particularly preferred amatoxins are α-amanitin, β-amanitin, and amaninamide, as well as salts, chemical derivatives, semisynthetic analogs, and synthetic analogs thereof.

In a preferred embodiment of the second aspect the amatoxin is connected to the antibody or, if present, to the linker L2 via the δC-atom of amatoxin amino acid 3 (see Fig. 1). In preferred amatoxins usable in the present invention said amino acid 3 is isoleucine, γ-hydroxy-isoleucine or γ,δ-dihydroxy-isoleucine.

In preferred embodiments of the second aspect, the amatoxin is connected to the antibody or, if present, to the linker L2 via an oxygen atom bound to the δC-atom of amatoxin amino acid 3. It is further preferred that the amatoxin is connected to the antibody or, if present, to the linker L2 via an ester linkage, an ether linkage or a urethane linkage. In these embodiments, it is preferred that amino acid 3 is γ,δ-dihydroxy-isoleucine.

In preferred embodiments of the second aspect, the antibody is connected to the amatoxin or, if present, to the linker L2 via an amino group present in the antibody.

In a preferred embodiment of the second aspect the linker L2 is an alkyl, heteroalkyl, alkenyl, heteroalkenyl, alkynyl, heteroalkynyl, cycloalkyl, heterocycloalkyl, aryl, heteroaryl, aralkyl, or a heteroaralkyl group, optionally substituted. In further preferred embodiments of the fifth aspect the linker L2 comprises a disulfide bond.

In a third aspect the present invention is directed to the conjugate of the second aspect for use in medicine.

In a fourth aspect the present invention is directed to the conjugate of the second aspect for the treatment of cancer in a patient, wherein the cancer is selected from the group consisting of pancreatic cancer, cholangiocarcinoma, breast cancer and colorectal cancer.

In a fifth aspect the present invention is directed to the conjugate of the second aspect for the preparation of a pharmaceutical composition for the treatment of cancer in a patient, wherein the cancer is selected from the group consisting of pancreatic cancer, cholangiocarcinoma, breast cancer and colorectal cancer.

In a fifth aspect the present invention relates to a target-binding moiety toxin conjugate comprising: (i) a target-binding moiety; (ii) an amatoxin; and (iii) optionally a linker L3; wherein the amatoxin is connected to the target-binding moiety or, if present, to the linker L3 via the amatoxin amino acid 3, preferably the δ C-atom of amatoxin amino acid 3 (see Fig. 1). In preferred amatoxins usable in the present invention said amino acid 3 is isoleucine, γ-hydroxy-isoleucine or γ,8-dihydroxy-isoleucine.

In a preferred embodiment of the fifth aspect the amatoxin is connected to the target-binding moiety or, if present, to the linker L3 via an oxygen atom bound to the δC-atom of amatoxin amino acid 3. It is further preferred that the amatoxin is connected to the target-binding moiety or, if present, to the linker L3 via an ester linkage, preferably in the form of an amatoxin-O-C(O)-L3-target-binding moiety or an amatoxin-O-C(O)-target-binding moiety, more preferably an amatoxin-δC-O-C(O)-L3-target-binding moiety or an amatoxin-δC-O-C(O)-target-binding moiety and most preferably an amatoxin-δCH₂-O-C(O)-L3-target-binding moiety or an amatoxin-δCH₂-O-C(O)-target-binding moiety; an ether linkage, preferably in the form of an amatoxin-O-L3 or an amatoxin-O-target binding moiety, preferably an amatoxin-δC-O-L3-target binding moiety or an amatoxin-δC-O-target binding moiety, more preferably an amatoxin-δCH₂-O-L3-target binding moiety or an amatoxin-δCH₂-O-target binding moiety; or an urethane linkage preferably in the form of an amatoxin-O-C(O)-NH-L3-target-binding moiety or an amatoxin-O-C(O)-NH-target-binding moiety, preferably an amatoxin-δC-O-C(O)-NH-L3-target-binding moiety or an amatoxin-δC-O-C(O)-NH-target-binding moiety, i.e. an amatoxin-δCH₂-O-C(O)-NH-L3-target-binding moiety or an amatoxin-δCH₂-O-C(O)-NH-target-binding moiety. In these embodiments, it is preferred that amino acid 3 is γ,δ-dihydroxy-isoleucine.

In preferred embodiments of the fifth aspect the linker L3 is present and the conjugate has one of the following structures: (i) amatoxin-δC-O-C(O)-L3-C(O)-NH-target-binding moiety; (ii) amatoxin-δC-O-L3-C(O)-NH-target-binding moiety; or (iii) amatoxin-δC-O-C(O)-NH-L3-C(O)-NH-target-binding moiety, preferably (i) amatoxin-δCH₂-O-C(O)-L3-C(O)-NH-target-binding moiety; (ii) amatoxin-δCH₂-O-L3-C(O)-NH-target-binding moiety; or (iii) amatoxin-δCH₂-O-C(O)-NH-L3-C(O)-NH-target-binding moiety.

In a preferred embodiment of the fifth aspect the target-binding moiety is connected to the amatoxin or, if present, to the linker L3 via an amino group present in the target-binding moiety.

In a preferred embodiment of the fifth aspect the amatoxin is selected from α-amanitin, β-amanitin, γ-amanitin, ε-amanitin, amanin, amaninamide, amanullin, or amanullinic acid (all shown in Fig. 1), as well as salts, chemical derivatives, semisynthetic analogs, and synthetic analogs thereof. Particularly preferred amatoxins are α-amanitin, β-amanitin, and amaninamide, as well as salts, chemical derivatives, semisynthetic analogs, and synthetic analogs thereof.

In a preferred embodiment of the fifth aspect the linker L3 is an alkyl, heteroalkyl, alkenyl, heteroalkenyl, alkynyl, heteroalkynyl, cycloalkyl, heterocycloalkyl, aryl, heteroaryl, aralkyl, or a heteroaralkyl group, optionally substituted. In further preferred embodiments of the fifth aspect the linker L3 comprises a disulfide bond.

In a preferred embodiment of the fifth aspect the target-binding moiety specifically binds to an epitope that is present on a tumour cell. It is particularly preferred that the target-binding moiety specifically binds to an epitope of epithelial cell adhesion molecule (EpCAM).

In a preferred embodiment of the fifth aspect the target binding moiety is selected from the group consisting of: (i) antibody or antigen-binding fragment thereof; (ii) antibody-like protein; and (iii) nucleic acid aptamer. In a preferred embodiment the antibody or the antigen-binding fragment thereof is selected from a diabody, a tetrabody, a nanobody, a chimeric antibody, a deimmunized antibody, a humanized antibody or a human antibody. In a preferred embodiment the antigen binding fragment is selected from the group consisting of Fab, F(ab')₂, Fd, Fv, single-chain Fv, and disulfide-linked Fvs (dsFv). In a preferred embodiment the antibody or the antigen binding fragment thereof comprises (a) either the membrane-bound form of the heavy chain of huHEA125 (SEQ ID NO: 1) or the soluble form of the heavy chain of huHEA125 (SEQ ID NO: 2); and/or (b) the light chain of huHEA125 (SEQ ID NO: 11).

In an sixth aspect the present invention relates to a target-binding moiety toxin conjugate according to the fifth aspect for use in medicine.

In a seventh aspect the present invention relates to a target-binding moiety toxin conjugate according to the fifth aspect for the treatment of cancer in a patient, wherein the cancer is selected from the group consisting of pancreatic cancer, cholangiocarcinoma, breast cancer, colorectal cancer, lung cancer, prostate cancer, ovarian cancer, stomach cancer, kidney cancer, malignant melanoma, leukemia and malignant lymphoma.

In an eighth aspect the present invention is directed to a pharmaceutical composition comprising the antibody toxin conjugate of the first aspect or of the second aspect or the target-binding moiety toxin conjugate according to the fifth aspect and further comprising one or more pharmaceutically acceptable diluents, carriers, excipients, fillers, binders, lubricants, glidants, disintegrants, adsorbents; and/or preservatives.

The target binding moiety of the fifth to seventh embodiment is in preferred embodiments a protein, in particular an antibody. Proteins and in particular antibodies will comprise several amino acids, which allow the coupling of amatoxins. Preferred amino acids have free amino, hydroxy, or carbonyl-groups, including Lys, Gln, Glu, Asp, Asn, Thr, and Ser. Accordingly, it is possible to couple more than one amatoxin molecules to one protein molecule. An increase of the number of amatoxins per molecule will also increase the toxicity. Accordingly, in a preferred embodiment the ratio of antibody of the first to fourth embodiment and he target binding moiety of the fifth to seventh embodiment to amatoxin is between 1 protein molecule to between 1 and 15 amatoxin molecules, preferably 1, 2, 3, 4, 5, 6, 7, 8, 9, 10, 11, 12, 13, 14, or 15. For the purpose of the calculation of the ratio in case of dimmers like IgGs the dimmer is considered as one molecule. Similar ratios are preferred, if the target binding moiety is not a protein.

It is particularly preferred that the pharmaceutical composition of the eighth aspects can be used in the form of systemically administered medicaments. These include parenterals, which comprise among others injectables and infusions. Injectables are formulated either in the form of ampoules or as so called ready-for-use injectables, e.g. ready-to-use syringes or single-use syringes and aside from this in puncturable flasks for multiple withdrawal. The administration of injectables can be in the form of subcutaneous (s.c.), intramuscular (i.m.), intravenous (i.v.) or intracutaneous (i.c.) application. In particular, it is possible to produce the respectively suitable injection formulations as a suspension of crystals, solutions, nanoparticular or a colloid dispersed systems like, e.g. hydrosols.

Injectable formulations can further be produced as concentrates, which can be dissolved or dispersed with aqueous isotonic diluents. The infusion can also be prepared in form of isotonic solutions, fatty emulsions, liposomal formulations and micro-emulsions. Similar to injectables, infusion formulations can also be prepared in the form of concentrates for dilution. Injectable formulations can also be applied in the form of permanent infusions both in in-patient and ambulant therapy, e.g. by way of mini-pumps.

It is possible to add to parenteral drug formulations, for example, albumin, plasma, expander, surface-active substances, organic diluents, pH-influencing substances, complexing substances or polymeric substances, in particular as substances to influence the adsorption of the target-binding moiety toxin conjugates of the invention to proteins or polymers or they can also be added with the aim to reduce the adsorption of the target-binding moiety toxin conjugates of the invention to materials like injection instruments or packaging-materials, for example, plastic or glass.

The target-binding moiety toxin conjugates of the invention can be bound to microcarriers or nanoparticles in parenterals like, for example, to finely dispersed particles based on poly(meth)acrylates, polylactates, polyglycolates, polyamino acids or polyether urethanes. Parenteral formulations can also be modified as depot preparations, e.g. based on the "multiple unit principle", if the target-binding moiety toxin conjugates of the invention are introduced in finely dispersed, dispersed and suspended form, respectively, or as a suspension of crystals in the medicament or based on the "single unit principle" if the target-binding moiety toxin conjugate of the invention is enclosed in a formulation, e.g. in a tablet or a rod which is subsequently implanted. These implants or depot medicaments in single unit and multiple unit formulations often consist out of so called biodegradable polymers like e.g. polyesters of lactic and glycolic acid, polyether urethanes, polyamino acids, poly(meth)acrylates or polysaccharides.

Adjuvants and carriers added during the production of the pharmaceutical compositions of the present invention formulated as parenterals are preferably aqua sterilisata (sterilized water), pH value influencing substances like, e.g. organic or inorganic acids or bases as well as salts thereof, buffering substances for adjusting pH values, substances for isotonization like e.g. sodium chloride, sodium hydrogen carbonate, glucose and fructose, tensides and surfactants, respectively, and emulsifiers like, e.g. partial esters of fatty acids of polyoxyethylene sorbitans (for example, Tween^{®}) or, e.g. fatty acid esters of polyoxyethylenes (for example, Cremophor^{®}), fatty oils like, e.g. peanut oil, soybean oil or castor oil, synthetic esters of fatty acids like, e.g. ethyl oleate, isopropyl myristate and neutral oil (for example, Miglyol^{®}) as well as polymeric adjuvants like, e.g. gelatine, dextran, polyvinylpyrrolidone, additives which increase the solubility of organic solvents like, e.g. propylene glycol, ethanol, N,N-dimethylacetamide, propylene glycol or complex forming substances like, e.g. citrate and urea, preservatives like, e.g. benzoic acid hydroxypropyl ester and methyl ester, benzyl alcohol, antioxidants like e.g. sodium sulfite and stabilizers like e.g. EDTA.

When formulating the pharmaceutical compositions of the present invention as suspensions in a preferred embodiment thickening agents to prevent the setting of the target-binding moiety toxin conjugates of the invention or, tensides and polyelectrolytes to assure the resuspendability of sediments and/or complex forming agents like, for example, EDTA are added. It is also possible to achieve complexes of the active ingredient with various polymers. Examples of such polymers are polyethylene glycol, polystyrol, carboxymethyl cellulose, Pluronics^{®} or polyethylene glycol sorbit fatty acid ester. The target-binding moiety toxin conjugates of the invention can also be incorporated in liquid formulations in the form of inclusion compounds e.g. with cyclodextrins. In particular embodiments dispersing agents can be added as further adjuvants. For the production of lyophilisates scaffolding agents like mannite, dextran, saccharose, human albumin, lactose, PVP or varieties of gelatine can be used.

In a further aspect the present invention is directed to a method of treating pancreatic cancer, cholangiocarcinoma, or colorectal cancer in a patient in need thereof, comprising administering to the patient an effective amount of an antibody toxin conjugate as defined in the first aspect.

In a further aspect the present invention is directed to antibody toxin conjugates as defined in the claims for use in a method of treating pancreatic cancer, cholangiocarcinoma, breast cancer or colorectal cancer in a patient in need thereof, comprising administering to the patient an effective amount of an antibody toxin conjugate as defined in the third aspect.

In a further aspect the present invention is directed to antibody toxin conjugates as defined in the claims for use in a method of treating pancreatic cancer, cholangiocarcinoma, breast cancer or colorectal cancer in a patient in need thereof, comprising administering to the patient an effective amount of an target-binding moiety toxin conjugate as defined in the fifth aspect.

### EXAMPLES

In the following, the invention is explained in more detail by examples:

### Example 1: Comparison of binding affinities to target cells between antibody huHEA125 and antibody toxin conjugate amanitin-huHEA125

### 1.1 Chimeric antibody huHEA125

Several years ago, the inventors have established a hybridoma cell line secreting the anti-EpCAM mouse monoclonal antibody HEA125 (Moldenhauer et al., 1987; Momburg et al., 1987). Using molecular biology techniques this hybridoma line was reconstructed to produce a chimeric version of the antibody consisting of the mouse variable domains hooked up to human kappa constant light chain and human IgG1 constant heavy chain. The resulting antibody huHEA125 binds to EpCAM-expressing cells with high affinity (K_{d} = 2.2x10⁻⁹ M) and high specificity. The gene sequence and the amino acid sequence of huHEA125 immunoglobulin are shown below:

### huHEA125 heavy chain

Peptide sequence heavy chain, membrane bound form (IGHV/IGHD/IGHJ/IGHG1; IGHG1 is underlined) (SEQ ID NO: 1):

Peptide sequence heavy chain, secreted form (SEQ ID NO: 2):

Peptide sequence (IGHV/IGHD/IGHJ = VH domain; the framework regions FR1, FR2, FR3 and FR4 are underlined (SEQ ID NO: 3):

Nucleic acid sequence (annotated according to the IMGT-nomenclature, IGHV/IGHD/IGHJ; IGHD underlined; IGHJ doubly underlined):
FR1 (SEQ ID NO: 4):
CDR1 (SEQ ID NO: 5):
   GGATTCGATTTTAGTAGATTCTGG
FR2 (SEQ ID NO: 6):
   ATGACTTGGGTCCGGCAGGCTCCAGGGAAAGGGCTAGAATGGATTGGAGAA
CDR2 (SEQ ID NO: 7):
   ATTAATCTAGATAGCAGTACGATA
FR3 (SEQ ID NO: 8):
CDR3 (SEQ ID NO: 9):
   TCAAGAGGTATTTCTATGGACTAC
FR4 (SEQ ID NO: 10):
   TGGGGTCAGGGAACCTCAGTCACCGTCTCCTCA

### huHEA 125 light chain

Peptide sequence light chain (IGKV/IGKJ/IGKC; IGKC is underlined) (SEQ ID NO: 11):

Peptide sequence (IGKV/IGKJ = VL domain; the framework regions FR1, FR2, FR3 and FR4 are underlined) (SEQ ID NO: 12):

Nucleic acid sequence (annotated according to the IMGT-nomenclature, IGKV/IGKJ; IGKV is underlined; IGKJ is doubly underlined):
FR1 (SEQ ID NO: 13):
CDR1 (SEQ ID NO: 14):
   CAGAGCATTGGCATAAGT
FR2 (SEQ ID NO: 15):
   TTACACTGGTATCAGCAAAGACCAAGTGATTCTCCAAGGCTTCTCATAAAG
CDR2 (SEQ 1D NO: 16):
   TATGCTTCT
FR3 (SEQ ID NO: 17):
CDR3 (SEQ ID NO: 18:
   CAACAAAGTAATATCTGGCCAACCACG
FR4 (SEQ ID NO: 19):
   TTCGGTGCTGGGACCAAGCTGGAGCTGAAA

### 1.2 Control antibody Xolair®

The control antibody Xolair® (Omalizumab, human IgG1 antibody directed against human IgE immunoglobulin) was produced by Novartis, Germany.

### 1.3 Synthesis of α-Amanitin Antibody Conjugate

### 13.1 Synthesis of α-Amanitin-glutarate

3.0 mg (3.3 µmol) of α-amanitin, dried *in vacuo* over P₄O₁₀ was dissolved in 0.25 ml of dry pyridine and reacted with 0.9 mg (79 µmol) glutaric anhydride in 0.1 ml pyridine for 24h at RT in the dark. The peptide was precipitated by addition of 7 ml of dry diethylether, centrifuged, and the solid washed a second time with diethylether and centrifuged.

By way of this reaction an α-amanitin derivative is obtained wherein R₁ = -OH (in Fig. 1) is replaced by R₁ = -O-C(O)-(CH₂)₃-COOH.

### 1.3.2 Synthesis of α-Amanitin-glutaric acid N-hydroxysuccinimidate

3.4 mg of α-amanitin glutarate (3.3 µmol) was dissolved in 0.05 ml of dry dimethylformamide (DMF), and 2.4 mg (7 eq.) of N-hydroxy-succinimide dissolved in 0.01 ml of DMF were added. After the addition of 1.2 mg of dicyclohexylcarbodiimide in 0.01 ml of DMF the reaction was allowed to proceed for 16 h at RT. The solution was separated from the crystals formed, and the peptide precipitated by the addition of 4 ml of dry diethylether. After centrifugation, the pellet was washed with another 4 ml of ether and centrifuged. The solid was dissolved in 0.1 ml of dimethylformamide and immediately used for the reaction with the antibody solution.

### 1.3.3 Synthesis of α-Amanitin-glutarate-huHEA125

0.1 ml of the solution of 3.0 mg of α-amanitin-glutaric acid N-hydroxysuccinimidate was added to 10 mg of hu-HEA125 antibody in 5 ml of PBS and reacted under slow rotation at 5°C in the dark. After 16h the solution was applied to a Sephadex G25 column (120 x 1.5 cm) equilibrated with PBS, and the protein fraction collected. Amanitin load was determined spectrophotometrically from the absorption difference at 310 nm of the protein solution against a blank containing the same concentration of the native antibody, using the molar extinction coefficient for amatoxins of 13.500 cm^{-1.}M⁻¹. Ratio α-amanitin: IgG of this preparation was ca. 8.

### 1.4 Binding Competition Analysis

Binding of amanitin-huHEA125 conjugate vs. non-conjugated huHEA125 antibody was analyzed in a competition experiment by flow cytometry. The α-amanitin-huHEA125 conjugate was synthesized as described above in sections 1.3.1 to 1.3.3.

Colo205 target cells (colon cancer metastasis) were washed twice in FACS buffer (Dulbecco's PBS with 1% heat-inactivated fetal calf serum and 0.1 % sodium azide) counted and adjusted to 2x10⁷ cells per ml. Fifty µl of cell suspension was given to each well of a 96 well U-bottom microtiter plate to which 50 µl/well of FITC-labeled huHEA125 antibody was pipetted. Serial dilutions of amanitin-huHEA125 or huHEA125 ranging from 400µg/ml to 10ng/ml final dilution were added in triplicates in a volume of 50 µl/well and incubated for 1 h on ice. Subsequently, the plate was centrifuged (2 min at 2000 rpm) and the supernatant was removed from the cells. Cells were re-suspended in 150 µl of FACS buffer and centrifuged again. After two washing steps by centrifugation, cells were taken up in 100 µl/well of propidium iodide solution (1 µg/ml in FACS buffer) allowing discrimination of dead cells. Analysis was performed on a FACScan cytometer (Becton and Dickinson, Heidelberg, Germany) using CellQuest software.

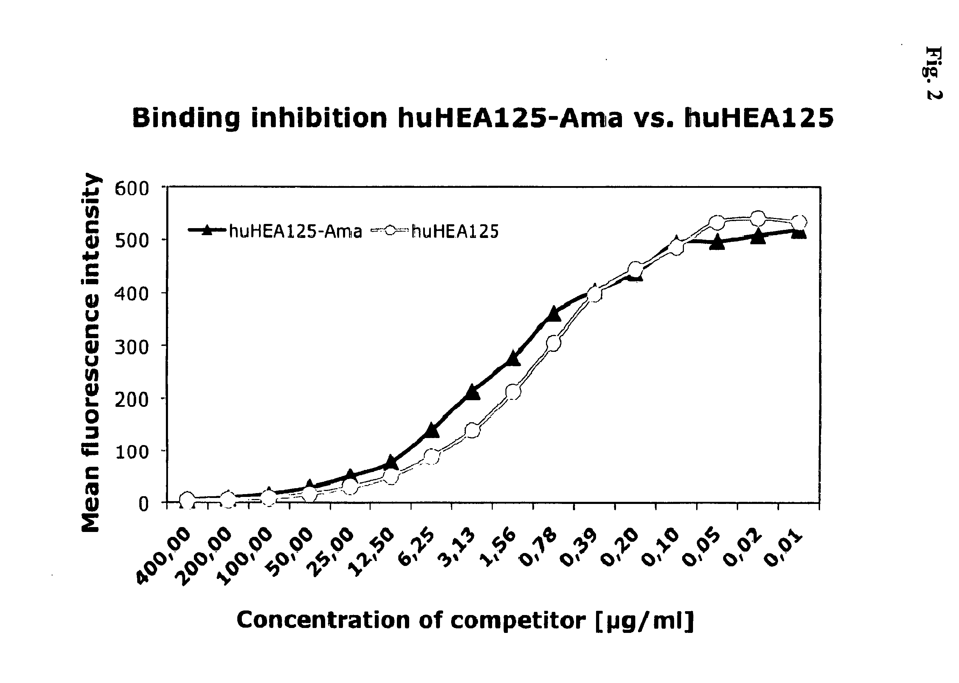

As shown in **Figure 2** competition of binding to target cells with increasing amounts of huHEA125-amanitin conjugate or unmodified huHEA125 antibody revealed a comparable binding strength over the whole concentration range from 10ng/ml to 400µg/ml competing antibody or antibody conjugate. Therefore, the conjugation procedure did not significantly alter the affinity of huHEA 25-amanitin to the target cells.

### Example 2: Surface expression of EpCAM antigen on various carcinoma cell lines detected by indirect immunofluorescence

Cell lines Capan-1, Colo205, OZ, MCF-7, BxPC-3 and PC-3 were first incubated with either huHEA125 or Xolair®. After washing, binding of the primary antibody was visualized by FITC-labelled F(ab')₂ goat anti-human IgG (H+L) as second step reagent. The results are shown in Fig. 3A (Capan-1), Fig. 3B (Colo205), Fig. 3C (OZ), Fig. 3D (MCF-7), Fig. 3E (BxPC-3), and Fig. 3F (PC-3). The grey-shaded histograms in the left side of each diagram show the results obtained with control antibody Xolair®; the histograms having a white area in the right side of each diagram show the results obtained with antibody huHEA125.

### Example 3: Induction of carcinoma cell proliferation inhibition by amanitin and amanitin/antibody conjugates

### 3.1 Carcinoma cell lines

The following carcinoma cell lines were used for growth inhibition studies:

| | |
|---|---|
| Capan-1, BxPC-3 | human pancreatic adenocarcinoma |
| MCF-7 | human breast adenocarcinoma |
| Colo205 | human colon cancer metastasis |
| OZ | human cholangiocarcinoma |
| PC-3 | human prostate adenocarcinoma |

### 3.2 Proliferation inhibition assay

Inhibition of cell growth by amanitin-IgG conjugates was determined by incorporation of [³H]-thymidine. Serial dilutions of amanitin-huHEA125, amanitin-Xolair and free amanitin in complete medium (RPMI 1640 supplemented with 10 % heat-inactivated FCS, 2 mM L-glutamine and 1 mM sodium pyruvate) ranging from 2x10⁻⁵ M to 6x10-¹³ were prepared in triplicates in a volume of 100 µl in the wells of a 96 well flat-bottom tissue culture microtiter plate. Cells were added in a volume of 50 µl per well at a density of 2x10⁴ per ml. Plates were incubated in a humidified atmosphere at 37 °C and 5 % CO₂ for 72 or 96 h. At 20 h before the end of the assay, 1 µCi of [³H]-thymidine was added. Subsequently plates were processed with a Tomtec cell harvester and the incorporated radioactivity was determined by liquid scintillation counting (Wallac Betaplate Liquid Scintillation Counter, PerkinElmer Life and Analytical Sciences) and given as cpm.

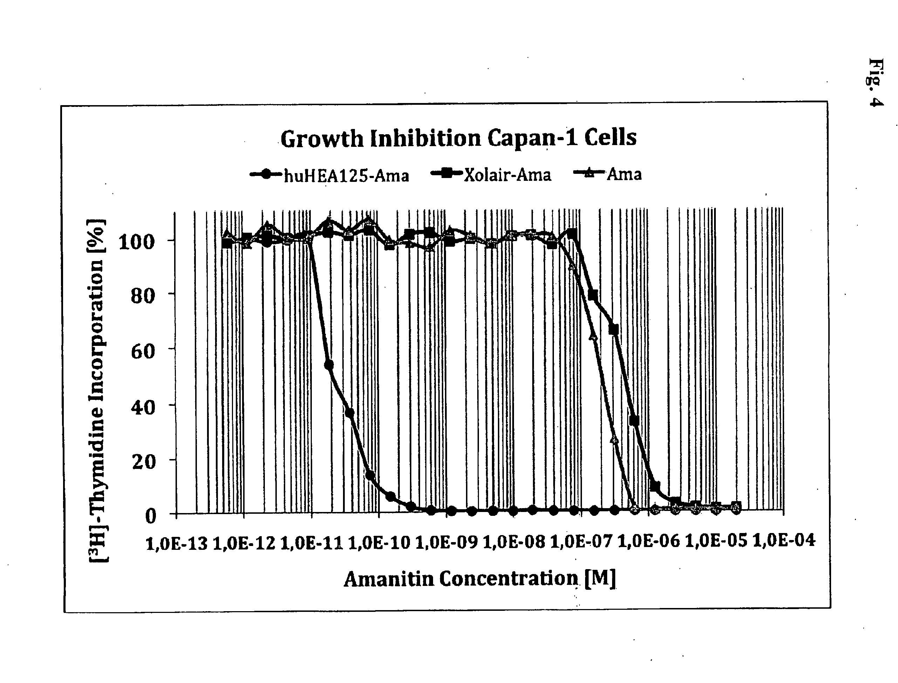

In case of the pancreatic carcinoma cell line Capan-1 the huHEA125-amanitin immunotoxin induced growth arrest at amanitin concentrations of 1x10⁻¹¹ to 3x10⁻¹⁰ M as depicted in Figure 4.

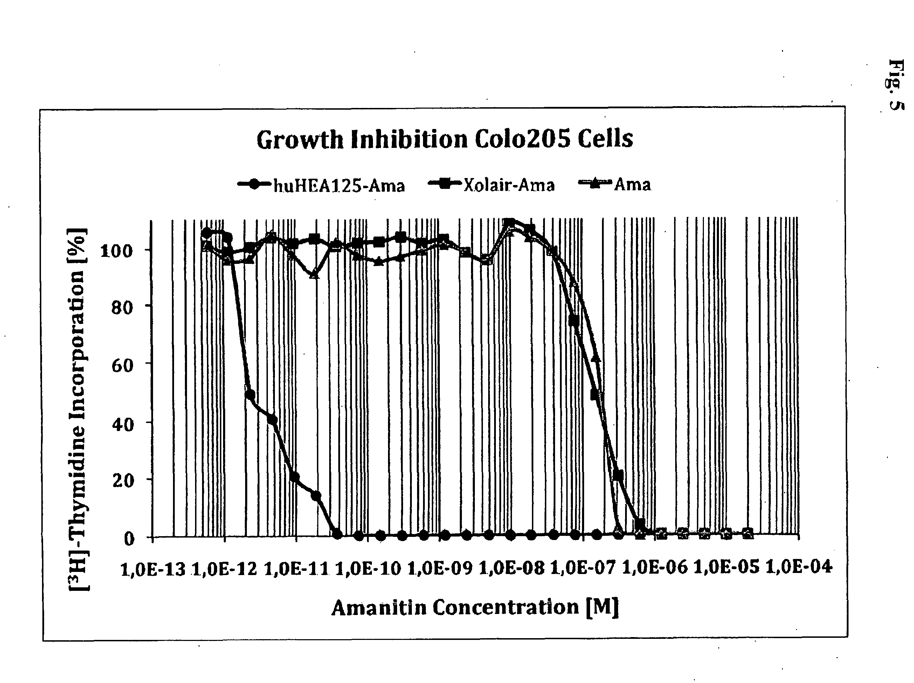

In case of the colon cancer cell line Colo205 the huHEA125-amanitin immunotoxin induced growth arrest at amanitin concentrations of 1x10⁻¹² to 4x10⁻¹¹ M as depicted in Figure 5.

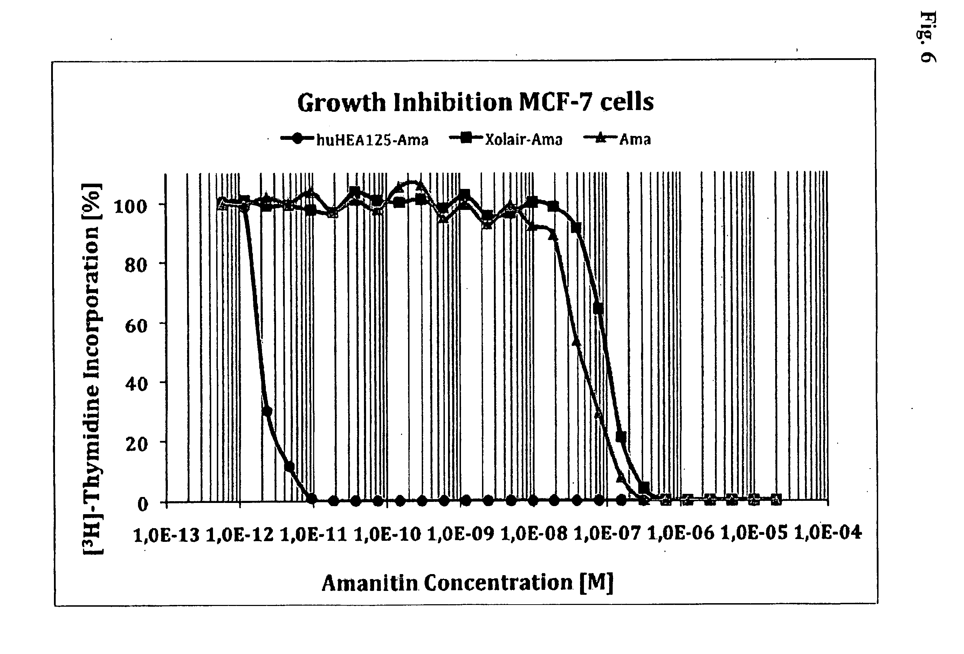

In case of the breast cancer cell line MCF-7 the huHEA125-amanitin immunotoxin induced growth arrest at amanitin concentrations of 1x10⁻¹² to 1x10⁻¹¹ M as depicted in Figure 6.

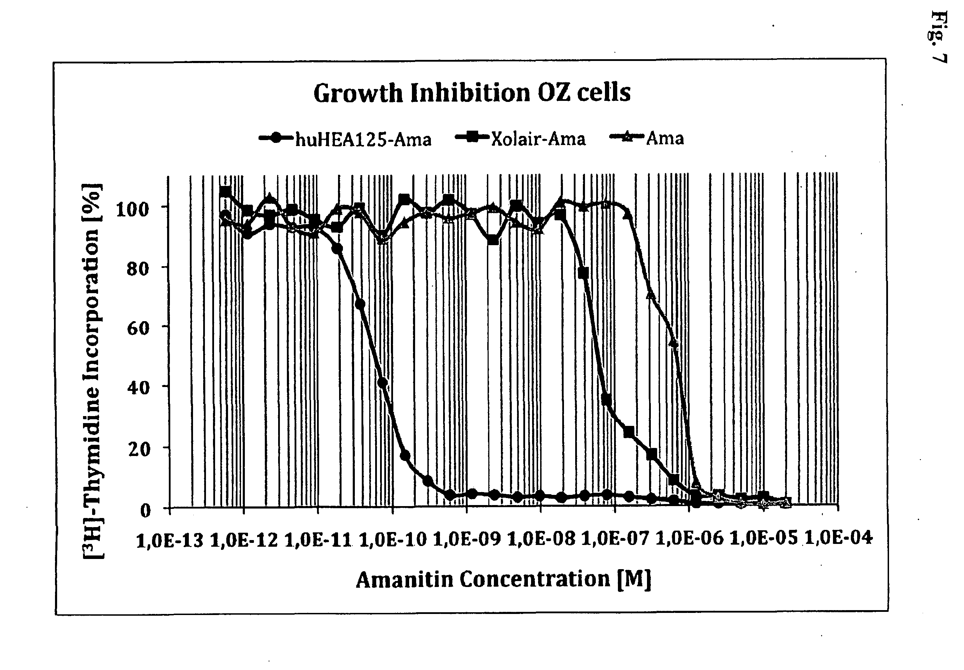

In case of the cholangiocarcinoma cell line OZ the huHEA125-amanitin immunotoxin induced growth arrest at amanitin concentrations of 1x10⁻¹¹ to 6x10⁻¹⁰ M as depicted in Figure 7.

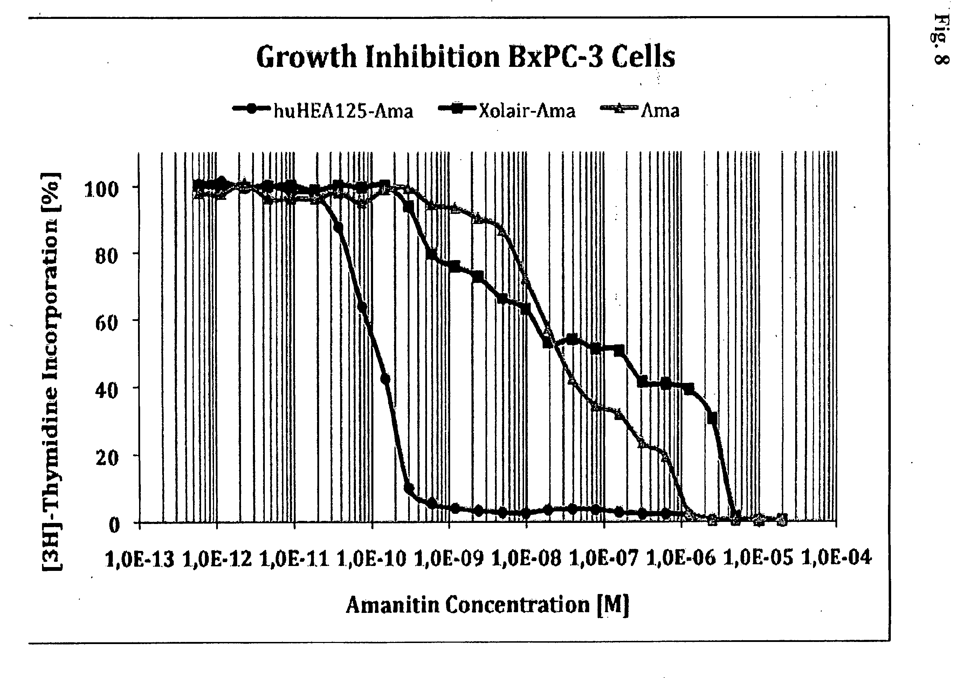

In case of the pancreatic cell line BxPC-3 the huHEA125-amanitin immunotoxin induced growth arrest at amanitin concentrations of 2x10⁻¹¹ to 6x10⁻¹⁰ M as depicted in Figure 8.

### Example 4: Inhibition of tumor growth in vivo by amanitin/antibody conjugate using two xenograft mouse tumor models

Five- to six-week old immunodeficient NOD/SCID mice were used for all experiments. BxPC-3 pancreatic or PC-3 prostate tumor cells (5x10⁶ in 100 µl PBS) were transplanted subcutaneously to the right flank of the mice. Ten days later, when BxPC-3 tumors reached a volume of 30-80 mm³ and PC-3 tumors reached a volume of 40-190 mm³, the treatment was initiated. Animals received either control huHEA125 mAb at a dose of 15 mg/kg or huHEA125-amanitin conjugate (huHEA125-Ama) at a dose of 50 µg/kg of amanitin. Antibody and conjugate were administered as a single intraperitoneal injection.

Tumor growth was monitored for 16 days after initiation of the treatment. Tumor size was measured externally every third day using a caliper. Tumor volume was calculated according to the formula: V = π/6*a*b*c, where a, b and c are diameters in three dimensions. Data are presented as a relative tumor size/volume increase from the time of antibody administration.

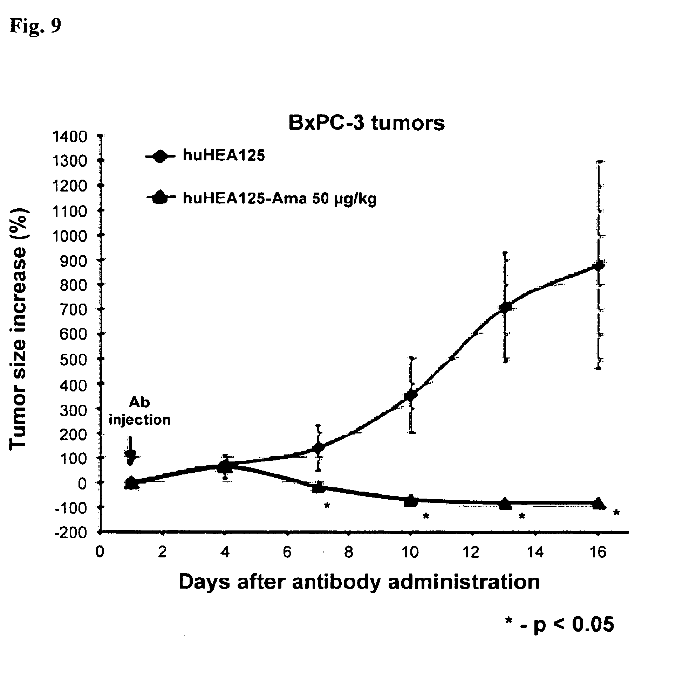

Administration of huHEA125-Ama at a dose of 50 µg/kg of amanitin was well tolerated by BxPC-3 tumor bearing mice (n = 6). There was neither a decrease in body weight of the mice nor an elevation of liver enzymes (LDH, ALT, AST and AP was measured in the serum on the last day of experiment). The tumor growth was strongly inhibited by this dose of conjugate. All mice responded to treatment and tumor volume regressed dramatically starting from day 7 after the administration of conjugate. At the end of follow-up on day 16, tumor was completely eradicated in 50% of the mice. In contrast, in control mice that received non-conjugated huHEA125 mAb tumor volume increased by approximately 880% (Fig. 9).

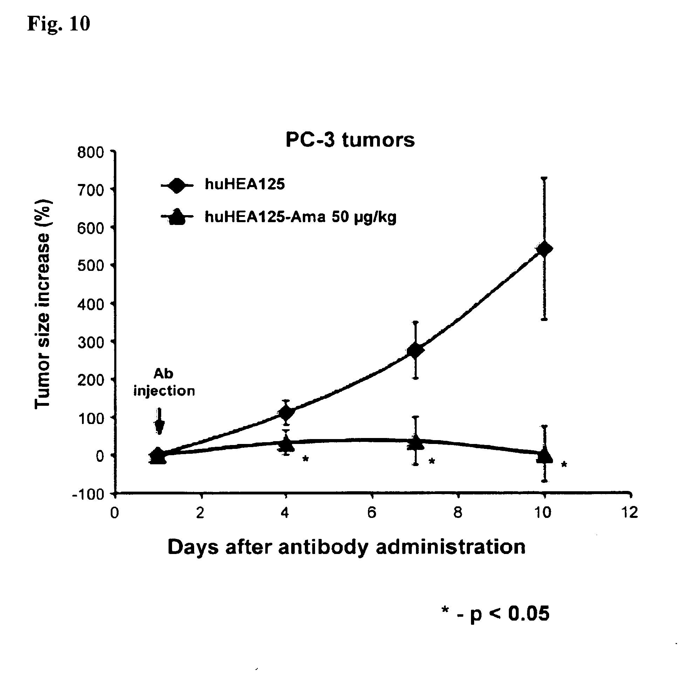

In case of PC-3 tumor bearing mice huHEA125-Ama at a dose of 50 µg/kg of amanitin was well tolerated. No decrease in the body weight of the mice was observed. The tumor growth was strongly retarded by this dose of the conjugate. Ten days after huHEA125-Ama administration, the tumor volume was similar to that at the initiation of the treatment. In contrast, in control mice that received non-conjugated huHEA125 mAb tumor volume increased by approximately 550%. The experiment was terminated on day 10 after treatment due to the large size of tumors in the control group (Fig. 10).

### REFERENCES

Al-Hajj M., Wicha M.S., Benito-Hernandez A., Morrison S.J., Clarke M.F. Prospective identification of tumorigenic breast cancer cells. Proc. Natl. Acad. Sci. USA 100(7), 3983-3988 (2003)
Allard W.J., Matera J., Miller M.C., Repollet M., Connelly M.C., Rao C., Tibbe A.G., Uhr J.W., Terstappen L.W. Tumor cells circulate in the peripheral blood of all major carcinomas but not in healthy subjects or patients with nonmalignant diseases. Clin. Cancer Res. 10(20), 6897-6904 (2004)
Baeuerle P.A. and Gires O. EpCAM (CD326) finding its role in cancer. Br. J. Cancer 96(3), 417-423 (2007)
Balzar M., Winter M.J., de Boer C.J., Litvinov S.V. The biology of the 17-1A antigen (Ep-CAM). J. Mol. Med. 77(10), 699-712 (1999)
Binz H.K., Amstutz P., Plückthun A. Engineering novel binding proteins from nonimmunoglobulin domains. Nat Biotechnol. 23(10):1257-1268 (2005)
Brody E.N. and Gold L., Aptamers as therapeutic and diagnostic agents. J. Biotechnol. 74(1):5-13 (2000)
Dalerba P., Dylla S.J., Park I.K., Liu R., Wang X., Cho R.W., Hoey T., Gurney A., Huang E.H., Simeone D.M., Shelton A.A., Parmiani G., Castelli C., Clarke M.F. Phenotypic characterization of human colorectal cancer stem cells. Proc. Natl. Acad. Sci. USA 104(24), 10158-10163 (2007)
Gastl G., Spizzo G., Obrist P., Dunser M., Mikuz G. Ep-CAM overexpression in breast cancer as a predictor of survival. Lancet 356(9246), 1981-1982 (2000)
Holliger P., Prospero T., Winter G. "Diabodies": small bivalent and bispecific antibody fragments. Proc. Natl. Acad. Sci. U.S.A. 90(14), 6444-6448 (1993)
Leuenberger, H.G.W, Nagel, B. and Kölbl, H. eds. "A multilingual glossary of biotechnological terms: (IUPAC Recommendations)", Helvetica Chimica Acta, CH-4010 Basel, Switzerland), 1995
Li C., Heidt D.G., Dalerba P., Burant C.F., Zhang L., Adsay V., Wicha M., Clarke M.F., Simeone D.M. Identification of pancreatic cancer stem cells. Cancer Res. 67(3), 1030-1037 (2007)
Moldenhauer G., Momburg F., Möller P., Schwartz R., Hämmerling G.J. Epithelium-specific surface glycoprotein of Mr 34,000 is a widely distributed human carcinoma marker. Br. J. Cancer 56(6), 714-721 (1987)
Momburg F., Moldenhauer G., Hammerling G.J., Möller P. Immunohistochemical study of the expression of a Mr 34,000 human epithelium-specific surface glycoprotein in normal and malignant tissues. Cancer Res. 47(11), 2883-2891 (1987)
Nagrath S., Sequist L.V., Maheswaran S., Bell D.W., Irimia D., Ulkus L., Smith M.R., Kwak E.L., Digumarthy S., Muzikansky A., Ryan P., Balis U.J., Tompkins R.G., Haber D.A., Toner M. Isolation of rare circulating tumour cells in cancer patients by microchip technology. Nature 450(7173), 1235-1239 (2007)
Spizzo G., Obrist P., Ensinger C., Theurl I., Dünser M., Ramoni A., Gunsilius E., Eibl G., Mikuz G., Gastl G. Prognostic significance of Ep-CAM AND Her-2/neu overexpression in invasive breast cancer. Int. J. Cancer 98(6), 883-888 (2002)
Spizzo G., Went P., Dirnhofer S., Obrist P., Simon R., Spichtin H., Maurer R., Metzger U., von Castelberg B., Bart R., Stopatschinskaya S., Köchli O.R., Haas P., Mross F., Zuber M., Dietrich H., Bischoff S., Mirlacher M., Sauter G., Gastl G. High Ep-CAM expression is associated with poor prognosis in node-positive breast cancer. Breast Cancer Res. Treat. 86(3), 207-213 (2004)
Trzpis M., McLaughlin P.M., de Leij L.M., Harmsen M.C. Epithelial cell adhesion molecule: more than a carcinoma marker and adhesion molecule. Am. J. Pathol. 171 (2), 386-395 (2007)
Varga M., Obrist P., Schneeberger S., Mühlmann G., Felgel-Famholz C., Fong D., Zitt M., Brunhuber T., Schäfer G., Gastl G., Spizzo G. Overexpression of epithelial cell adhesion molecule antigen in gallbladder carcinoma is an independent marker for poor survival. Clin. Cancer Res. 10(9), 3131-3136 (2004)
Went P.T., Lugli A., Meier S., Bundi M., Mirlacher M., Sauter G., Dirnhofer S. Frequent EpCam protein expression in human carcinomas. Hum. Pathol. 35(1), 122-128, 2004
Wieland, T. and Faulstich H. Amatoxins, phallotoxins, phallolysin, and antamanide: the biologically active components of poisonous Amanita mushrooms. CRC Crit. Rev. Biochem. 5(3), 185-260 (1978)
Winter M.J., Nagtegaal I.D., van Krieken J.H., Litvinov S.V. The epithelial cell adhesion molecule (Ep-CAM) as a morphoregulatory molecule is a tool in surgical pathology. Am. J. Pathol. 163(6), 2139-2148 (2003)

### SEQUENCE LISTING - FREE TEXT INFORMATION

| | |
|---|---|
| SEQ ID NO: 1: | chimeric antibody huHEA125, heavy chain, membrane-bound form |
| SEQ ID NO: 2: | chimeric antibody huHEA125, heavy chain, secreted form |
| SEQ ID NO: 3: | chimeric antibody huHEA125, heavy chain, VH domain |
| SEQ ID NO: 4: | chimeric antibody huHEA125, heavy chain, FR1 segment |
| SEQ ID NO: 5: | chimeric antibody huHEA125, heavy chain, CDR1 segment |
| SEQ ID NO: 6: | chimeric antibody huHEA125, heavy chain, FR2 segment |
| SEQ ID NO: 7: | chimeric antibody huHEA125, heavy chain, CDR2 segment |
| SEQ ID NO: 8: | chimeric antibody huHEA125, heavy chain, FR3 segment |
| SEQ ID NO: 9: | chimeric antibody huHEA125, heavy chain, CDR3 segment |
| SEQ ID NO: 10: | chimeric antibody huHEA125, heavy chain, FR4 segment |
| SEQ ID NO: 11: | chimeric antibody huHEA125, light chain |
| SEQ ID NO: 12: | chimeric antibody huHEA125, light chain, VL domain |
| SEQ ID NO: 13: | chimeric antibody huHEA125, light chain, FR1 segment |
| SEQ ID NO: 14: | chimeric antibody huHEA125, light chain, CDR1 segment |
| SEQ ID NO: 15: | chimeric antibody huHEA125, light chain, FR2 segment |
| SEQ ID NO: 16: | chimeric antibody huHEA125, light chain, CDR2 segment |
| SEQ ID NO: 17: | chimeric antibody huHEA125, light chain, FR3 segment |
| SEQ ID NO: 18: | chimeric antibody huHEA125, light chain, CDR3 segment |
| SEQ ID NO: 19: | chimeric antibody huHEA 125, light chain, FR4 segment |
| SEQ ID NO: 20: | chimeric antibody huHEA125, heavy chain, CDR1 domain |
| SEQ ID NO: 21: | chimeric antibody huHEA125, heavy chain, CDR2 domain |
| SEQ ID NO: 22: | chimeric antibody huHEA125, heavy chain, CDR3 domain |
| SEQ ID NO: 23: | chimeric antibody huHEA125, light chain, CDR1 domain |
| SEQ ID NO: 24: | chimeric antibody huHEA125, light chain, CDR2 domain |
| SEQ ID NO: 25: | chimeric antibody huHEA125, light chain, CDR3 domain |
| SEQ ID NO: 26: | chimeric antibody huHEA125, heavy chain, constant domain, membrane bound form |
| SEQ ID NO: 27: | chimeric antibody huHEA125, heavy chain, constant domain, secreted form |
| SEQ ID NO: 28: | chimeric antibody huHEA125, light chain, constant domain |

## Claims

1. An antibody toxin conjugate for use in the treatment of pancreatic cancer, cholangiocarcinoma, or colorectal cancer in a patient,
wherein the conjugate comprises
(i) an antibody or antigen binding fragment thereof specifically binding to an epitope of epithelial cell adhesion molecule (EpCAM), which is preferably an epitope of human EpCAM;
(ii) an amatoxin; and
(iii) optionally a linker L1;
wherein the antibody or antigen binding fragment thereof is preferably selected from a diabody, a tetrabody, a nanobody, a chimeric antibody, a deimmunized antibody, a humanized antibody or a human antibody, and/or
wherein the antigen binding fragment is preferably selected from the group consisting of Fab, F(ab')₂, Fd, Fv, single-chain Fv, and disulfide-linked Fvs (dsFv).

2. The conjugate for use according to claim 1, wherein the antibody or the antigen binding fragment thereof comprises
(a) the CDR3 domain (SEQ ID NO: 22) of the heavy chain of huHEA125; and/or
(b) the CDR3 domain (SEQ ID NO: 25) of the light chain of huHEA 125,
wherein the antibody or the antigen binding fragment thereof preferably additionally comprises one or more of the following:
(a1) the CDR2 domain (SEQ ID NO: 21) of the heavy chain of huHEA125;
(b1) the CDR1 domain (SEQ ID NO: 20) of the heavy chain of huHEA125;
(c1) the CDR2 domain (SEQ ID NO: 24) of the light chain of huHEA125; and
(d1) the CDR1 domain (SEQ ID NO: 23) of the light chain of huHEA125, and/or
wherein the antibody or the antigen binding fragment thereof preferably comprises the VH domain of huHEA125 (SEQ ID NO: 3) and/or the VL domain of huHEA125 (SEQ ID NO: 12), and/or
wherein the antibody or the antigen binding fragment thereof preferably comprises
(a2) either the membrane-bound form of the heavy chain of huHEA125 (SEQ ID NO: 1) or the soluble form of the heavy chain of huHEA125 (SEQ ID NO: 2); and/or
(b2) the light chain of huHEA125 (SEQ ID NO: 11).

3. The conjugate for use according to any one of claims 1 or 2, wherein the amatoxin is selected from α-amanitin, β-amanitin, γ-amanitin, ε-amanitin, amanin, amaninamide, amanullin, or amanullinic acid, or salts thereof or a compound structurally related to any one of α-amanitin, β-amanitin, γ-amanitin, ε-amanitin, amanin, amaninamide, amanullin, or amanullinic acid and that exhibits at least 1% of the inhibitory activity against mammalian RNA polymerase II as compared to at least one of α-amanitin, β-amanitin, γ-amanitin, ε-amanitin, amanin, amaninamide, amanullin, and amanullinic acid.

4. An antibody toxin conjugate comprising
(i) an antibody or an antigen binding fragment thereof specifically binding to epithelial cell adhesion molecule (EpCAM), wherein the antibody or an antigen binding fragment thereof comprises:
(a) the heavy chain of huHEA 125, wherein the heavy chain is selected from the group consisting of:
(a1) the membrane-bound form of the heavy chain according to SEQ ID NO: 1,
wherein the variable domain of the heavy chain VH as shown in SEQ ID NO: 3 comprises between 0 and 10 amino acid exchanges, between 0 and 10 amino acid deletions and/or between 0 and 10 amino acid additions positioned in the framework regions of VH, and
wherein the constant domain of the heavy chain as shown in SEQ ID NO: 26 comprises between 0 and 10 amino acid exchanges, between 0 and 10 amino acid deletions and/or between 0 and 10 amino acid additions; and
(a2) the soluble form of the heavy chain according to SEQ ID NO: 2, wherein the variable domain of the heavy chain VH as shown in SEQ ID NO: 3 comprises between 0 and 10 amino acid exchanges, between 0 and 10 amino acid deletions and/or between 0 and 10 amino acid additions positioned in the framework regions of VH, and
wherein the constant domain of the heavy chain as shown in SEQ ID NO: 27 comprises between 0 and 10 amino acid exchanges, between 0 and 10 amino acid deletions and/or between 0 and 10 amino acid additions;
and
(b) the light chain of huHEA125 according to SEQ ID NO: 11,
wherein the variable domain of the light chain VL as shown in SEQ ID NO: 12 comprises between 0 and 10 amino acid exchanges, between 0 and 10 amino acid deletions and/or between 0 and 10 amino acid additions positioned in the framework regions of VL, and
wherein the constant domain of the light chain CL as shown in SEQ ID NO: 28 comprises between 0 and 10 amino acid exchanges, between 0 and 10 amino acid deletions and/or between 0 and 10 amino acid additions.
(ii) an amatoxin; and
(iii) optionally a linker L2,
wherein the antibody or antigen binding fragment thereof is preferably selected from a chimeric antibody, a deimmunized antibody, a humanized antibody or a human antibody, and/or
wherein the antigen binding fragment is preferably selected from the group consisting of Fab, F(ab')₂, and Fd, and/or
wherein the antibody preferably is huHEA125 or an antigen binding fragment thereof.

5. The conjugate of claim 4, wherein the amatoxin is selected from α-amanitin, β-amanitin, γ-amanitin, ε-amanitin, amanin, amaninamide, amanullin, or amanullinic acid, or salts thereof or a compound structurally related to any one of α-amanitin, β-amanitin, γ-amanitin, ε-amanitin, amanin, amaninamide, amanullin, or amanullinic acid and that exhibits at least 1% of the inhibitory activity against mammalian RNA polymerase II as compared to at least one of α-amanitin, β-amanitin, γ-amanitin, ε-amanitin, amanin, amaninamide, amanullin, and amanullinic acid.

6. The conjugate of any one of claims 4 or 5 for use in medicine.

7. The conjugate of any one of claims 4 or 5 for use in the treatment of cancer in a patient, wherein the cancer is selected from the group consisting of pancreatic cancer, cholangiocarcinoma, breast cancer and colorectal cancer.

8. A target-binding moiety toxin conjugate comprising:
(i) a target-binding moiety specifically binding to an epitope of epithelial cell adhesion molecule (EpCAM),
(ii) an amatoxin; and
(iii) optionally a linker L3;
wherein the amatoxin is connected to the target-binding moiety or, if present, to the linker L3 via the δ C-atom of amatoxin amino acid 3, and wherein the target moiety is an antibody or antigen-binding fragment thereof, and
wherein the amatoxin is preferably connected to the target-binding moiety or, if present, to the linker L3 via an oxygen atom bound to the δ C-atom of amatoxin amino acid 3, and
wherein the amatoxin is preferably connected to the target-binding moiety or, if present, to the linker L3 via an ester linkage, an ether linkage or a urethane linkage, and/or
wherein the target-binding moiety is preferably connected to the amatoxin or, if present, to the linker L3 via an amino group present in the target-binding moiety.

9. The target-binding moiety toxin conjugate of claim 8, wherein the linker L3 is present and the conjugate has one of the following structures:
(i) amatoxin-δC-O-C(O)-NH-C(O)-NH-target-binding moiety;
(ii) amatoxin-δC-O-L3-C(O)-NH-target-binding moiety; or
(iii) amatoxin-δC-O-C(O)-NH-L3-C(O)-NH-target-binding moiety.

10. The target-binding moiety toxin conjugate of any one of claims 8 or 9, wherein the amatoxin is selected from α-amanitin, β-amanitin, γ-amanitin, ε-amanitin, amanin, amaninamide, amanullin, or amanullinic acid, or salts thereof or a compound structurally related to any one of α-amanitin, β-amanitin, γ-amanitin, ε-amanitin, amanin, amaninamide, amanullin, or amanullinic acid and that exhibits at least 1% of the inhibitory activity against mammalian RNA polymerase II as compared to at least one of α-amanitin, β-amanitin, γ-amanitin, ε-amanitin, amanin, amaninamide, amanullin, and amanullinic acid.

11. The target-binding moiety toxin conjugate of any one of claims 8 to 10, wherein the linker L3 is a alkyl, heteroalkyl, alkenyl, heteroalkenyl, alkynyl, heteroalkynyl, cycloalkyl, heterocycloalkyl, aryl, heteroaryl, aralkyl, or a heteroaralkyl group, optionally substituted, and
wherein the linker L3 preferably comprises a disulfide bond.

12. The target-binding moiety toxin conjugate of any one of claims 8 to 11, wherein the target-binding moiety specifically binds to an epitope that is present on a tumour cell.

13. The target-binding moiety toxin conjugate of any one of claims 8 to 12, wherein the antibody or the antigen-binding fragment thereof is selected from a diabody, a tetrabody, a nanobody, a chimeric antibody, a deimmunized antibody, a humanized antibody or a human antibody, and/or
wherein the antigen binding fragment is preferably selected from the group consisting of Fab, F(ab')₂, Fd, Fv, single-chain Fv, and disulfide-linked Fvs (dsFv), and/or
wherein the antibody or the antigen binding fragment thereof preferably comprises
(a) either the membrane-bound form of the heavy chain of huHEA125 (SEQ ID NO: 1) or the soluble form of the heavy chain of huHEA125 (SEQ ID NO: 2); and/or
(b) the light chain of huHEA 125 (SEQ ID NO: 11).

14. The target-binding moiety toxin conjugate of any one of claims 8 to 13 for use in medicine.

15. The target-binding moiety toxin conjugate of any one of claims 8 to 14 for use in the treatment of cancer in a patient, wherein the cancer is selected from the group consisting of pancreatic cancer, cholangiocarcinoma, breast cancer, colorectal cancer, lung cancer, prostate cancer, ovarian cancer, stomach cancer, kidney cancer, malignant melanoma, leukemia and malignant lymphoma.

16. Pharmaceutical composition comprising the antibody toxin conjugate for use according to claims 1-3 or pharmaceutical composition comprising the antibody toxin conjugate according to any one of claims 4 or 5 or the target-binding moiety toxin conjugate according to any one of claims 8 to 13 and further comprising one or more pharmaceutically acceptable diluents, carriers, excipients, fillers, binders, lubricants, glidants, disintegrants, adsorbents; and/or preservatives.

## Patentansprüche

1. Antikörper-Toxin-Konjugat zur Verwendung bei der Behandlung von Bauchspeicheldrüsenkrebs, Cholangiokarzinom, oder Kolorektalkrebs in einem Patienten, wobei das Konjugat umfasst
(i) einen Antikörper oder ein Antigen-bindendes Fragment davon, welcher/welches in spezifischer Weise an ein Epitop des epithelialen Zelladhäsionsmoleküls (EpCAM) bindet, welches vorzugsweise ein Epitop des humanen EpCAM ist;
(ii) ein Amatoxin; und
(iii) optional einen Linker L1;
wobei der Antikörper oder das Antigen-bindende Fragment davon vorzugsweise ausgewählt ist aus einem Diabody, einem Tetrabody, einem Nanobody, einem chimären Antikörper, einem deimmunisierten Antikörper, einem humanisierten Antikörper oder einem humanen Antikörper, und/oder
wobei das Antigen-bindende Fragment vorzugsweise ausgewählt ist aus der Gruppe bestehend aus Fab, F(ab')₂, Fd, Fv, einkettigem Fv und Disulfid-verknüpften Fvs (dsFv).

2. Konjugat zur Verwendung gemäß Anspruch 1, wobei der Antikörper oder das Antigen-bindende Fragment davon umfasst
(a) die CDR3-Domäne (SEQ ID NO: 22) der schweren Kette von huHEA125; und/oder
(b) die CDR3-Domäne (SEQ ID NO: 25) der leichten Kette von huHEA125,
wobei der Antikörper oder das Antigen-bindende Fragment davon vorzugsweise zusätzlich eine oder mehrere der folgenden umfasst:
(a1) die CDR2-Domäne (SEQ ID NO: 21) der schweren Kette von huHEA125;
(b1) die CDR1-Domäne (SEQ ID NO: 20) der schweren Kette von huHEA125;
(c1) die CDR2-Domäne (SEQ ID NO: 24) der leichten Kette von huHEA125; und
(d1) die CDR1-Domäne (SEQ ID NO: 23) der leichten Kette von
huHEA125, und/oder
wobei der Antikörper oder das Antigen-bindende Fragment davon vorzugsweise die VH-Domäne von huHEA125 (SEQ ID NO: 3) und/oder die VL-Domäne von huHEA125 (SEQ ID NO: 12) umfasst, und/oder
wobei der Antikörper oder das Antigen-bindende Fragment davon vorzugsweise umfasst
(a2) entweder die membrangebundene Form der schweren Kette von huHEA125 (SEQ ID NO: 1) oder die lösliche Form der schweren Kette von huHEA125 (SEQ ID NO: 2); und/oder
(b2) die leichte Kette von huHEA125 (SEQ ID NO: 11).

3. Konjugat zur Verwendung gemäß einem der Ansprüche 1 oder 2, wobei das Amatoxin ausgewählt ist aus α-Amanitin, β-Amanitin, γ-Amanitin, ε-Amanitin, Amanin, Amaninamid, Amanullin oder Amanullinsäure, oder Salzen davon oder einer Verbindung, die strukturell mit einem von α-Amanitin, β-Amanitin, γ-Amanitin, ε-Amanitin, Amanin, Amaninamid, Amanullin oder Amanullinsäure verwandt ist und die mindestens 1% der inhibitorischen Aktivität gegen die Säugetier-RNA-Polymerase II im Vergleich zu wenigstens einem von α-Amanitin, β-Amanitin, γ-Amanitin, ε-Amanitin, Amanin, Amaninamid, Amanullin und Amanullinsäure aufweist.

4. Antikörper-Toxin-Konjugat umfassend
(i) einen Antikörper oder ein Antigen-bindendes Fragment davon, welcher/welches in spezifischer Weise das epitheliale Zelladhäsionsmolekül (EpCAM) bindet, wobei der Antikörper oder ein Antigen-bindendes Fragment davon umfasst:
(a) die schwere Kette von huHEA125, wobei die schwere Kette ausgewählt ist aus der Gruppe bestehend aus:
(a1) der membrangebundenen Form der schweren Kette gemäß SEQ ID NO: 1,
wobei der variable Bereich der schweren Kette VH, der in SEQ ID NO: 3 gezeigt ist, zwischen 0 und 10 Aminosäureaustauschen, zwischen 0 und 10 Aminosäuredeletionen und/oder zwischen 0 und 10 Aminosäureadditionen umfasst, die in den Gerüstregionen von VH positioniert sind, und
wobei der konstante Bereich der schweren Kette, der in SEQ ID NO: 26 gezeigt ist, zwischen 0 und 10 Aminosäureaustauschen, zwischen 0 und 10 Aminosäuredeletionen und/oder zwischen 0 und 10 Aminosäureadditionen umfasst; und
(a2) der löslichen Form der schweren Kette gemäß SEQ ID NO: 2,
wobei der variable Bereich der schweren Kette VH, der in SEQ ID NO: 3 gezeigt ist, zwischen 0 und 10 Aminosäureaustauschen, zwischen 0 und 10 Aminosäuredeletionen und/oder zwischen 0 und 10 Aminosäureadditionen umfasst, die in den Gerüstregionen von VH positioniert sind, und
wobei der konstante Bereich der schweren Kette, der in SEQ ID NO: 27 gezeigt ist, zwischen 0 und 10 Aminosäureaustauschen, zwischen 0 und 10 Aminosäuredeletionen und/oder zwischen 0 und 10 Aminosäureadditionen umfasst; und
(b) die leichte Kette von huHEA125 gemäß SEQ ID NO: 11, wobei der variable Bereich der leichten Kette VL, der in SEQ ID NO: 12 gezeigt ist, zwischen 0 und 10 Aminosäureaustauschen, zwischen 0 und 10 Aminosäuredeletionen und/oder zwischen 0 und 10 Aminosäureadditionen umfasst, die in den Gerüstregionen von VL positioniert sind, und
wobei der konstante Bereich der leichten Kette CL, der in SEQ ID NO: 28 gezeigt ist, zwischen 0 und 10 Aminosäureaustauschen, zwischen 0 und 10 Aminosäuredeletionen und/oder zwischen 0 und 10 Aminosäureadditionen umfasst,
(ii) ein Amatoxin; und
(iii) optional einen Linker L2,
wobei der Antikörper oder das Antigen-bindende Fragment davon vorzugsweise ausgewählt ist aus einem chimären Antikörper, einem deimmunisierten Antikörper, einem humanisierten Antikörper oder einem humanen Antikörper, und/oder
wobei das Antigen-bindende Fragment vorzugsweise ausgewählt ist aus der Gruppe bestehend aus Fab, F(ab')₂ und Fd, und/oder
wobei der Antikörper vorzugsweise ein huHEA125 oder ein Antigen-bindendes Fragment davon ist.

5. Konjugat nach Anspruch 4, wobei das Amatoxin ausgewählt ist aus α-Amanitin, β-Amanitin, γ-Amanitin, ε-Amanitin, Amanin, Amaninamid, Amanullin oder Amanullinsäure, oder Salzen davon oder einer Verbindung, die strukturell mit einem von α-Amanitin, β-Amanitin, γ-Amanitin, ε-Amanitin, Amanin, Amaninamid, Amanullin oder Amanullinsäure verwandt ist und die mindestens 1% der inhibitorischen Aktivität gegen die Säugetier-RNA-Polymerase II im Vergleich zu wenigstens einem von α-Amanitin, β-Amanitin, γ-Amanitin, ε-Amanitin, Amanin, Amaninamid, Amanullin und Amanullinsäure aufweist.

6. Konjugat nach einem der Ansprüche 4 oder 5 zur Verwendung in der Medizin.

7. Konjugat nach einem der Ansprüche 4 oder 5 zur Verwendung bei der Behandlung von Krebs in einem Patienten, wobei der Krebs ausgewählt ist aus der Gruppe bestehend aus Bauchspeicheldrüsenkrebs, Cholangiokarzinom, Brustkrebs und Kolorektalkrebs.

8. Ziel-bindende-Gruppe-Toxin-Konjugat umfassend:
(i) eine Ziel-bindende Gruppe, welche in spezifischer Weise ein Epitop des epithelialen Zelladhäsionsmoleküls (EpCAM) bindet,
(ii) ein Amatoxin; und
(iii) optional einen Linker L3;
wobei das Amatoxin mit der Ziel-bindenden Gruppe oder, falls vorhanden, mit dem Linker L3 über das δ C-Atom der dritten Aminosäure von Amatoxin in Verbindung steht und wobei die Ziel-bindende Gruppe ein Antikörper oder ein Antigen-bindendes Fragment davon ist, und
wobei das Amatoxin vorzugsweise mit der Ziel-bindenden Gruppe oder, falls vorhanden, mit dem Linker L3 über ein Sauerstoffatom, welches an das δ C-Atom der dritten Aminosäure von Amatoxin gebunden ist, in Verbindung steht, und
wobei das Amatoxin vorzugsweise mit der Ziel-bindenden Gruppe oder, falls vorhanden, mit dem Linker L3 über eine Ester-Verbindung, eine Ether-Verbindung oder eine Urethan-Verbindung verknüpft ist, und/oder
wobei die Ziel-bindende Gruppe vorzugsweise mit dem Amatoxin oder, falls vorhanden, mit dem Linker L3 über eine Aminogruppe, die in der Ziel-bindenden Gruppe vorhanden ist, verknüpft ist.

9. Ziel-bindende-Gruppe-Toxin-Konjugat nach Anspruch 8, wobei der Linker L3 vorhanden ist und das Konjugat eine der folgenden Strukturen aufweist:
(i) Amatoxin-δC-O-C(O)-L3-C(O)-NH-Ziel-bindende Gruppe;
(ii) Amatoxin-δC-O-L3-C(O)-NH-Ziel-bindende Gruppe; oder
(iii) Amatoxin-δC-O-C(O)-NH-L3-C(O)-NH-Ziel-bindende Gruppe.

10. Ziel-bindende-Gruppe-Toxin-Konjugat nach einem der Ansprüche 8 oder 9, wobei das Amatoxin ausgewählt ist aus α-Amanitin, β-Amanitin, γ-Amanitin, ε-Amanitin, Amanin, Amaninamid, Amanullin oder Amanullinsäure, oder Salzen davon oder einer Verbindung, die strukturell mit einem von α-Amanitin, β-Amanitin, γ-Amanitin, ε-Amanitin, Amanin, Amaninamid, Amanullin oder Amanullinsäure verwandt ist und die mindestens 1% der inhibitorischen Aktivität gegen die Säugetier-RNA-Polymerase II im Vergleich zu wenigstens einem von α-Amanitin, β-Amanitin, γ-Amanitin, ε-Amanitin, Amanin, Amaninamid, Amanullin und Amanullinsäure aufweist.

11. Ziel-bindende-Gruppe-Toxin-Konjugat nach einem der Ansprüche 8 bis 10, wobei der Linker L3 eine Alkyl-, Heteroalkyl-, Alkenyl-, Heteroalkenyl-, Alkinyl-, Heteroalkinyl-, Cycloalkyl-, Heterocycloalkyl-, Aryl-, Heteroaryl-, Aralkyl- oder eine Heteroaralkylgruppe ist, die optional substituiert ist, und wobei der Linker L3 vorzugsweise eine Disulfidbindung umfasst.

12. Ziel-bindende-Gruppe-Toxin-Konjugat nach einem der Ansprüche 8 bis 11, wobei die Ziel-bindende Gruppe in spezifischer Weise ein Epitop, das auf einer Tumorzelle vorhanden ist, bindet.

13. Ziel-bindende-Gruppe-Toxin-Konjugat nach einem der Ansprüche 8 bis 12, wobei der Antikörper oder das Antigen-bindende Fragment davon ausgewählt ist aus einem Diabody, einem Tetrabody, einem Nanobody, einem chimären Antikörper, einem deimmunisierten Antikörper, einem humanisierten Antikörper oder einem humanen Antikörper und/oder
wobei das Antigen-bindende Fragment vorzugsweise ausgewählt ist aus der Gruppe bestehend aus Fab, F(ab')₂, Fd, Fv, einkettigem Fv, und Disulfid-verknüpften Fvs (dsFv) und/oder
wobei der Antikörper oder das Antigen-bindende Fragment davon vorzugsweise umfasst
(a) entweder die membrangebundene Form der schweren Kette von huHEA125 (SEQ ID NO: 1) oder die lösliche Form der schweren Kette von huHEA125 (SEQ ID NO: 2); und/oder
(b) die leichte Kette von huHEA125 (SEQ ID NO: 11).

14. Ziel-bindende-Gruppe-Toxin-Konjugat nach einem der Ansprüche 8 bis 13 zur Verwendung in der Medizin.

15. Ziel-bindende-Gruppe-Toxin-Konjugat nach einem der Ansprüche 8 bis 14 zur Verwendung bei der Behandlung von Krebs in einem Patienten, wobei der Krebs ausgewählt ist aus der Gruppe bestehend aus Bauchspeicheldrüsenkrebs, Cholangiokarzinom, Brustkrebs, Kolorektalkrebs, Lungenkrebs, Prostatakrebs, Eierstockkrebs, Magenkrebs, Nierenkrebs, malignem Melanom, Leukämie und malignem Lymphom.

16. Pharmazeutische Zusammensetzung, die das Antikörper-Toxin-Konjugat zur Verwendung gemäß den Ansprüchen 1 bis 3 umfasst, oder pharmazeutische Zusammensetzung, die das Antikörper-Toxin-Konjugat gemäß einem der Ansprüche 4 oder 5 oder das Ziel-bindende-Gruppe-Toxin-Konjugat gemäß einem der Ansprüche 8 bis 13 umfasst und, die zudem ein oder mehrere pharmazeutisch verträgliche Verdünnungsmittel, Trägerstoffe, Hilfsstoffe, Füllstoffe, Bindemittel, Schmiermittel, Fließreguliermittel, Sprengmittel, Adsorptionsmittel und/oder Konservierungsstoffe umfasst.

## Revendications

1. Conjugué d'anticorps et de toxine pour utilisation dans le traitement du cancer du pancréas, du cholangiocarcinome, ou du cancer colorectal chez un patient,
dans lequel le conjugué comprend
(i) un anticorps ou un fragment de celui-ci se liant à un antigène, se liant spécifiquement à un épitope de molécule d'adhésion aux cellules épithéliales (EpCAM), qui est de préférence un épitope de EpCAM humaine;
(ii) une amatoxine; et
(iii) en option un lieur L1;
tandis que l'anticorps ou le fragment de celui-ci se liant à un antigène est de préférence choisi parmi un diacorps, un tétracorps, un nanocorps, un anticorps chimérique, un anticorps désimmunisé, un anticorps humanisé ou un anticorps humain, et/ou tandis que le fragment se liant à un antigène est de préférence choisi dans le groupe consistant en Fab, F(ab')₂, Fd, Fv, Fv à chaîne unique, et Fvs à liaison disulfure (dsFv).

2. Conjugué pour utilisation selon la revendication 1, dans lequel l'anticorps ou le fragment de celui-ci se liant à un antigène comprend
(a) le domaine CDR3 (SEQ ID NO: 22) de la chaîne lourde de huHEA125; et/ ou
(b) le domaine CDR3 (SEQ ID NO: 25) de la chaîne légère de huHEA125,
tandis que l'anticorps ou le fragment de celui-ci se liant à un antigène comprend en outre de préférence un ou plusieurs des suivants:
(a1) le domaine CDR2 (SEQ ID NO: 21) de la chaîne lourde de huHEA125;
(b1) le domaine CDR1 (SEQ ID NO: 20) de la chaîne lourde de huHEA125;
(c1) le domaine CDR2 (SEQ ID NO: 24) de la chaîne légère de huHEA125; et
(d1) le domaine CDR1 (SEQ ID NO: 23) de la chaîne légère de huHEA125, et/ ou
tandis que l'anticorps ou le fragment de celui-ci se liant à un antigène comprend de préférence le domaine VH de huHEA125 (SEQ ID NO: 3) et/ou le domaine VL de huHEA125 (SEQ ID NO: 12), et/ou tandis que l'anticorps ou le fragment de celui-ci se liant à un antigène comprend de préférence
(a2) soit la forme liée à une membrane de la chaîne lourde de huHEA125 (SEQ ID NO: 1) soit la forme soluble de la chaîne lourde de huHEA125 (SEQ ID NO: 2); et/ou
(b2) la chaîne légère de huHEA125 (SEQ ID NO: 11).

3. Conjugué pour utilisation selon l'une quelconque des revendications 1 ou 2, dans lequel l'amatoxine est choisie parmi α-amanitine, β-amanitine, γ-amanitine, ε-amanitine, amanine, amaninamide, amanulline, ou acide amanullinique, ou leurs sels ou un composé structurellement apparenté à l'une quelconque des α-amanitine, β-amanitine, γ-amanitine, ε-amanitine, amanine, amaninamide, amanulline, ou acide amanullinique et qui présente au moins 1 % de l'activité inhibitrice contre l'ARN polymérase II mammalienne par comparaison avec au moins l'un des α-amanitine, β-amanitine, γ-amanitine, ε-amanitine, amanine, amaninamide, amanulline, et acide amanullinique.

4. Conjugué d'anticorps et de toxine comprenant
(i) un anticorps ou un fragment de celui-ci se liant à un antigène, se liant spécifiquement à la molécule d'adhésion aux cellules épithéliales (EpCAM), tandis que l'anticorps ou le fragment de celui-ci se liant à un antigène comprend:
(a) la chaîne lourde de huHEA125, tandis que la chaîne lourde est choisie dans le groupe consistant en:
(a1) la forme liée à une membrane de la chaîne lourde selon SEQ ID NO:1,
tandis que le domaine variable du VH de chaîne lourde tel que représenté dans SEQ ID NO: 3 comprend entre 0 et 10 échanges d'acides aminés, entre 0 et 10 délétions d'acides aminés et/ou entre 0 et 10 additions d'acides aminés situés dans les régions de structure de VH, et
tandis que le domaine constant de la chaîne lourde tel que représenté dans SEQ ID NO: 26 comprend entre 0 et 10 échanges d'acides aminés, entre 0 et 10 délétions d'acides et/ou entre 0 et 10 additions d'acides aminés; et
(a2) la forme soluble de la chaîne lourde selon SEQ ID NO: 2, tandis que le domaine variable de la chaîne lourde du VH de chaîne lourde tel que représenté dans SEQ ID NO: 3 comprend entre 0 et 10 échanges d'acides aminés, entre 0 et 10 délétions d'acides aminés et/ou entre 0 et 10 additions d'acides aminés disposés dans les régions de structure de VH, et
dans lequel le domaine constant de la chaîne lourde tel que représenté dans SEQ ID NO: 27 comprend entre 0 et 10 échanges d'acides aminés, entre 0 et 10 délétions d'acides aminés et/ou entre 0 et 10 additions d'acides aminés;
et
(b) la chaîne légère de huHEA125 selon SEQ ID NO: 11,
tandis que le domaine variable de la chaîne légère VL tel que représenté dans SEQ ID NO: 12 comprend entre 0 et 10 échanges d'acides aminés, entre 0 et 10 délétions d'acides aminés et/ou entre 0 et 10 additions d'acides aminés disposés dans les régions de structure de VL, et
tandis que le domaine constant de la chaîne légère CL tel que représenté dans SEQ ID NO: 28 comprend entre 0 et 10 échanges d'acides aminés, entre 0 et 10 délétions d'acides aminés et/ou entre 0 et 10 additions d'acides aminés,
(ii) une amatoxine; et
(iii) en option un lieur L2,
tandis que l'anticorps ou le fragment de celui-ci se liant à un antigène est de préférence choisi parmi un anticorps chimérique, un anticorps désimmunisé, un anticorps humanisé ou un anticorps humain, et/ou
tandis que le fragment se liant à un antigène est de préférence choisi dans le groupe consistant en Fab, F(ab')₂, et Fd, et/ou tandis que l'anticorps est de préférence huHEA125 ou un fragment de celui-ci se liant à un antigène.

5. Conjugué selon la revendication 4, dans lequel l'amatoxine est choisie parmi: α-amanitine, β-amanitine, γ-amanitine, ε-amanitine, amanine, amaninamide, amanulline, ou acide amanullinique, ou leurs sels ou un composé structurellement apparenté à l'une quelconque des α-amanitine, β-amanitine, γ-amanitine, ε-amanitine, amanine, amaninamide, amanulline, ou acide amanullinique et qui présente au moins 1% de l'activité inhibitrice contre l'ARN polymérase II mammalienne par comparaison avec au moins l'un des α-amanitine, β-amanitine, γ-amanitine, ε-amanitine, amanine, amaninamide, amanulline, et acide amanullinique.

6. Conjugué selon l'une quelconque des revendications 4 ou 5 pour utilisation en médecine.

7. Conjugué selon l'une quelconque des revendications 4 ou 5 pour utilisation dans le traitement du cancer chez un patient, dans lequel le cancer est choisi dans le groupe consistant en cancer du pancréas, cholangiocarcinome, cancer du sein et cancer colorectal.

8. Conjugué d'entité se liant à une cible et de toxine, comprenant:
(i) une entité se liant à une cible, se liant spécifiquement à un épitope de molécule d'adhésion aux cellules épithéliales (EpCAM),
(ii) une amatoxine; et
(iii) en option un lieur L3;
tandis que l'amatoxine est reliée à l'entité se liant à une cible ou, s'il est présent, au lieur L3 via l'atome C en δ de l'acide aminé 3 de l'amatoxine, et tandis que l'entité cible est un anticorps ou un fragment de celui-ci se liant à un antigène, et
tandis que l'amatoxine est de préférence reliée à l'entité se liant à une cible ou, s'il est présent, au lieur L3 via un atome d'oxygène lié à l'atome C en δ de l'acide aminé 3 de l'amatoxine, et
tandis que l'amatoxine est de préférence liée à l'entité se liant à une cible ou, s'il est présent, au lieur L3 via une liaison ester, une liaison éther ou une liaison uréthane, et/ou
tandis que l'entité se liant à une cible est de préférence reliée à l'amatoxine ou, s'il est présent, au lieur L3 via un groupe amino présent dans l'entité se liant à une cible.

9. Conjugué d'entité se liant à une cible et de toxine selon la revendication 8, dans lequel le lieur L3 est présent et le conjugué a l'une des structures suivantes:
(i) amatoxine-δC-O-C(O)-L3-C(O)-NH- (entité se liant à une cible);
(ii) amatoxine-δC-O- L3-C(O)-NH- (entité se liant à une cible); ou
(iii) amatoxine-δC-O-C(O)-NH-L3-C(O)-NH- (entité se liant à une cible).

10. Conjugué d'entité se liant à une cible et de toxine selon l'une quelconque des revendications 8 ou 9, dans lequel l'amatoxine est choisie parmi α-amanitine, β-amanitine, γ-amanitine, ε-amanitine, amanine, amaninamide, amanulline, ou acide amanullinique, ou leurs sels ou un composé structurellement apparenté à l'une quelconque des α-amanitine, β-amanitine, γ-amanitine, ε-amanitine, amanine, amaninamide, amanulline, ou acide amanullinique et qui présente au moins 1% de l'activité inhibitrice contre l'ARN polymérase II mammalienne par comparaison avec au moins l'un des α-amanitine, β-amanitine, γ-amanitine, ε-amanitine, amanine, amaninamide, amanulline, et acide amanullinique.

11. Conjugué d'entité se liant à une cible et de toxine selon l'une quelconque des revendications 8 à 10, dans lequel le lieur L3 est un groupe alkyle, hétéroalkyle, alcényle, hétéroalcényle, alcynyle, hétéroalcynyle, cycloalkyle, hétérocycloalkyle, aryle, hétéroaryle, aralkyle, ou hétéroaralkyle, éventuellement substitué, et
dans lequel le lieur L3 comprend de préférence une liaison disulfure.

12. Conjugué d'entité se liant à une cible et de toxine selon l'une quelconque des revendications 8 à 11, dans lequel l'entité se liant à une cible se lie spécifiquement à un épitope qui est présent sur une cellule tumorale.

13. Conjugué d'entité se liant à une cible et de toxine selon l'une quelconque des revendications 8 à 12, dans lequel l'anticorps ou le fragment de celui-ci se liant à un antigène est choisi parmi un diacorps, un tétracorps, un nanocorps, un anticorps chimérique, un anticorps désimmunisé, un anticorps humanisé ou un anticorps humain, et/ ou
dans lequel le fragment se liant à un antigène est de préférence choisi dans le groupe consistant en Fab, F(ab')2, Fd, Fv, Fv à chaîne unique, et Fvs à liaison disulfure (dsFv), et/ou
tandis que l'anticorps ou le fragment de celui-ci se liant à un antigène comprend de préférence
(a) soit la forme liée à une membrane de la chaîne lourde de huHEA125 (SEQ ID NO: 1) soit la forme soluble de la chaîne lourde de huHEA125 (SEQ ID NO: 2); et/ ou
(b) la chaîne légère de huHEA125 (SEQ ID NO: 11).

14. Conjugué d'entité se liant à une cible et de toxine selon l'une quelconque des revendications 8 à 13 pour utilisation en médecine.

15. Conjugué d'entité se liant à une cible et de toxine selon l'une quelconque des revendications 8 à 14 pour utilisation dans le traitement du cancer chez un patient, tandis que le cancer est choisi dans le groupe consistant en cancer du pancréas, cholangiocarcinome, cancer du sein, cancer colorectal, cancer du poumon, cancer de la prostate, cancer de l'ovaire, cancer de l'estomac, cancer du rein, mélanome malin, leucémie et lymphome malin.

16. Composition pharmaceutique comprenant le conjugué d'anticorps et de toxine pour utilisation selon les revendications 1-3 ou composition pharmaceutique comprenant le conjugué d'anticorps et de toxine selon l'une quelconque des revendications 4 ou 5 ou le conjugué d'entité se liant à une cible et de toxine selon l'une quelconque des revendications 8 à 13, et comprenant en outre un ou plusieurs diluants, supports, excipients, charges, liants, lubrifiants, agents de glissement, agents de désintégration, adsorbants; et/ou conservateurs pharmaceutiquement acceptables.


### SEQUENCE LISTING

<110> Faulstich, Heinz Deutsches Krebsforschungszentrum
<120> AMATOXIN-ARMED TARGET-BINDING MOIETIES FOR THE TREATMENT OF CANCER
<130> 616-4 PCT
<140>
   <141> 2010-04-08
<150> US 61/167,690
   <151> 2009-04-08
<160> 28
<170> PatentIn version 3.3
<210> 1
   <211> 514
   <212> PRT
   <213> Artificial
<220>
   <223> chimeric antibody huHEA125, heavy chain, membrane-bound form
<400> 1
   Glu Val Lys Leu Leu Glu Ser Gly Gly Gly Leu Val Gln Pro Gly Gly
   1               5                   10                  15
   Ser Leu Lys Leu Ser Cys Ala Ala Ser Gly Phe Asp Phe Ser Arg Phe
               20                  25                  30
   Trp Met Thr Trp Val Arg Gln Ala Pro Gly Lys Gly Leu Glu Trp Ile
           35                  40                  45
   Gly Glu Ile Asn Leu Asp Ser Ser Thr Ile Asn Tyr Thr Pro Ser Leu
       50                  55                  60
   Lys Asp Lys Phe Ile Ile Ser Arg Asp Asn Ala Lys Asn Thr Leu Phe
   65                  70                  75                  80
   Leu Gln Met Ser Lys Val Arg Ser Glu Asp Thr Ala Leu Tyr Tyr Cys
                   85                  90                  95
   Ser Arg Gly Ile Ser Met Asp Tyr Trp Gly Gln Gly Thr Ser Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Cys Asp Lys Thr His Thr Cys
       210                 215                 220
   Pro Pro Cys Pro Ala Pro Glu Leu Leu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Lys
               260                 265                 270
   Phe Asn Trp Tyr Val Asp Gly Val Glu Val His Asn Ala Lys Thr Lys
           275                 280                 285
   Pro Arg Glu Glu Gln Tyr Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Ala Leu Pro Ala Pro Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Arg Asp Glu Leu Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Lys Leu Thr Val Asp Lys Ser Arg Trp Gln
                   405                 410                 415
   Gln Gly Asn Val Phe Ser Cys Ser Val Met His Glu Ala Leu His Asn
               420                 425                 430
   His Tyr Thr Gln Lys Ser Leu Ser Leu Ser Pro Gly Leu Gln Leu Asp
           435                 440                 445
   Glu Thr Cys Ala Glu Ala Gln Asp Gly Glu Leu Asp Gly Leu Trp Thr
       450                 455                 460
   Thr Ile Thr Ile Phe Ile Ser Leu Phe Leu Leu Ser Val Cys Tyr Ser
   465                 470                 475                 480
   Ala Ala Val Thr Leu Phe Lys Val Lys Trp Ile Phe Ser Ser Val Val
                   485                 490                 495
   Glu Leu Lys Gln Thr Leu Val Pro Glu Tyr Lys Asn Met Ile Gly Gln
               500                 505                 510
   Ala Pro
<210> 2
   <211> 445
   <212> PRT
   <213> Artificial
<220>
   <223> chimeric antibody huHEA125, heavy chain, secreted form
<400> 2
   Glu Val Lys Leu Leu Glu Ser Gly Gly Gly Leu Val Gln Pro Gly Gly
   1               5                   10                  15
   Ser Leu Lys Leu Ser Cys Ala Ala Ser Gly Phe Asp Phe Ser Arg Phe
               20                  25                  30
   Trp Met Thr Trp Val Arg Gln Ala Pro Gly Lys Gly Leu Glu Trp Ile
           35                  40                  45
   Gly Glu Ile Asn Leu Asp Ser Ser Thr Ile Asn Tyr Thr Pro Ser Leu
       50                  55                  60
   Lys Asp Lys Phe Ile Ile Ser Arg Asp Asn Ala Lys Asn Thr Leu Phe
   65                  70                  75                  80
   Leu Gln Met Ser Lys Val Arg Ser Glu Asp Thr Ala Leu Tyr Tyr Cys
                   85                  90                  95
   Ser Arg Gly Ile Ser Met Asp Tyr Trp Gly Gln Gly Thr Ser Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
   130 135 140
   Lys Asp Tyr Phe Pro Glu Pro Val Thr Val Ser Trp Asn Ser Gly Ala
   145 150 155 160
   Leu Thr Ser Gly Val His Thr Phe Pro Ala Val Leu Gln Ser Ser Gly
   165 170 175
   Leu Tyr Ser Leu Ser Ser Val Val Thr Val Pro Ser Ser Ser Leu Gly
   180 185 190
   Thr Gln Thr Tyr Ile Cys Asn Val Asn His Lys Pro Ser Asn Thr Lys
   195 200 205
   Val Asp Lys Lys Val Glu Pro Lys Ser Cys Asp Lys Thr His Thr Cys
   210 215 220
   Pro Pro Cys Pro Ala Pro Glu Leu Leu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser His Glu Asp Pro Glu Val Lys
   260 265 270
   Phe Asn Trp Tyr Val Asp Gly Val Glu Val His Asn Ala Lys Thr Lys
   275 280 285
   Pro Arg Glu Glu Gln Tyr Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Pro Ala Pro Ile Glu Lys Thr Ile Ser Lys
   325 330 335
   Ala Lys Gly Gln Pro Arg Glu Pro Gln Val Tyr Thr Leu Pro Pro Ser
   340 345 350
   Arg Asp Glu Leu Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
   435 440 445
<210> 3
   <211> 115
   <212> PRT
   <213> Artificial
<220>
   <223> chimeric antibody huHEA125, heavy chain, vH domain
<400> 3
   Glu Val Lys Leu Leu Glu Ser Gly Gly Gly Leu Val Gln Pro Gly Gly
   1 5 10 15
   Ser Leu Lys Leu Ser Cys Ala Ala Ser Gly Phe Asp Phe Ser Arg Phe
   20 25 30
   Trp Met Thr Trp Val Arg Gln Ala Pro Gly Lys Gly Leu Glu Trp Ile
   35 40 45
   Gly Glu Ile Asn Leu Asp Ser Ser Thr Ile Asn Tyr Thr Pro Ser Leu
   50 55 60
   Lys Asp Lys Phe Ile Ile Ser Arg Asp Asn Ala Lys Asn Thr Leu Phe
   65 70 75 80
   Leu Gln Met Ser Lys Val Arg Ser Glu Asp Thr Ala Leu Tyr Tyr Cys
   85 90 95
   Ser Arg Gly Ile Ser Met Asp Tyr Trp Gly Gln Gly Thr Ser Val Thr
   100 105 110
   Val Ser Ser
   115
<210> 4
   <211> 75
   <212> DNA
   <213> Artificial
<220>
   <223> chimeric antibody huHEA125, heavy chain, FR1 segment
<400> 4
   gaagtgaagc ttctcgagtc tggaggtggc ctggtgcagc ctggaggatc cctgaaactc 60
   tcctgtgcag cctca 75
<210> 5
   <211> 24
   <212> DNA
   <213> Artificial
<220>
   <223> chimeric antibody huHEA125, heavy chain, CDR1 segment
<400> 5
   ggattcgatt ttagtagatt ctgg 24
<210> 6
   <211> 51
   <212> DNA
   <213> Artificial
<220>
   <223> chimeric antibody huHEA125, heavy chain, FR2 segment
<400> 6
   atgacttggg tccggcaggc tccagggaaa gggctagaat ggattggaga a 51
<210> 7
   <211> 24
   <212> DNA
   <213> Artificial
<220>
   <223> chimeric antibody huHEA125, heavy chain, CDR2 segment
<400> 7
   attaatctag atagcagtac gata 24
<210> 8
   <211> 114
   <212> DNA
   <213> Artificial
<220>
   <223> chimeric antibody huHEA125, heavy chain, FR3 segment
<400> 8
   aactatacgc catctctaaa ggataaattc atcatctcca gggacaacgc caaaaatacg 60
   ctgttcctgc aaatgagcaa agtgagatct gaggacacag ccctttatta ctgt 114
<210> 9
   <211> 24
   <212> DNA
   <213> Artificial
<220>
   <223> chimeric antibody huHEA125, heavy chain, CDR3 segment
<400> 9
   tcaagaggta tttctatgga ctac 24
<210> 10
   <211> 33
   <212> DNA
   <213> Artificial
<220>
   <223> chimeric antibody huHEA125, heavy chain, FR4 segment
<400> 10
   tggggtcagg gaacctcagt caccgtctcc tca 33
<210> 11
   <211> 214
   <212> PRT
   <213> Artificial
<220>
   <223> chimeric antibody huHEA125, light chain
<400> 11
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Ser Ile Gly Ile Ser
               20                  25                  30
   Leu His Trp Tyr Gln Gln Arg Pro Ser Asp Ser Pro Arg Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Ser Ile Asn Ser Val Glu Ser
   65                  70                  75                  80
   Glu Asp Ile Ala Asp Tyr Tyr Cys Gln Gln Ser Asn Ile Trp Pro Thr
                   85                  90                  95
   Thr Phe Gly Ala Gly Thr Lys Leu Glu Leu Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 12
   <211> 107
   <212> PRT
   <213> Artificial
<220>
   <223> chimeric antibody huHEA125, light chain, VL domain
<400> 12
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Ser Ile Gly Ile Ser
               20                  25                  30
   Leu His Trp Tyr Gln Gln Arg Pro Ser Asp Ser Pro Arg Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Ser Ile Asn Ser Val Glu Ser
   65                  70                  75                  80
   Glu Asp Ile Ala Asp Tyr Tyr Cys Gln Gln Ser Asn Ile Trp Pro Thr
                   85                  90                  95
   Thr Phe Gly Ala Gly Thr Lys Leu Glu Leu Lys
               100                 105
<210> 13
   <211> 78
   <212> DNA
   <213> Artificial
<220>
   <223> chimeric antibody huHEA125, light chain, FR1 segment
<400> 13
   gacatcttgc tgactcagtc tccagccatc ctgtctgtga gtccaggaga aagagtcagt 60
   ttctcctgca gggccagt 78
<210> 14
   <211> 18
   <212> DNA
   <213> Artificial
<220>
   <223> chimeric antibody huHEA125, light chain, CDR1 segment
<400> 14
   cagagcattg gcataagt 18
<210> 15
   <211> 51
   <212> DNA
   <213> Artificial
<220>
   <223> chimeric antibody huHEA125, light chain, FR2 segment
<400> 15
   ttacactggt atcagcaaag accaagtgat tctccaaggc ttctcataaa g 51
<210> 16
   <211> 9
   <212> DNA
   <213> Artificial
<220>
   <223> chimeric antibody huHEA125, light chain, CDR2 segment
<400> 16
   tatgcttct 9
<210> 17
   <211> 108
   <212> DNA
   <213> Artificial
<220>
   <223> chimeric antibody huHEA125, light chain, FR3 segment
<400> 17
   gagtcaatct ctgggatccc ttccaggttt agtggcagtg gatcagggac agattttact 60
   cttagcatca acagtgtgga gtctgaagat attgcagatt attactgt 108
<210> 18
   <211> 27
   <212> DNA
   <213> Artificial
<220>
   <223> chimeric antibody huHEA125, light chain, CDR3 segment
<400> 18
   caacaaagta atatctggcc aaccacg 27
<210> 19
   <211> 30
   <212> DNA
   <213> Artificial
<220>
   <223> chimeric antibody huHEA125, light chain, FR4 segment
<400> 19
   ttcggtgctg ggaccaagct ggagctgaaa 30
<210> 20
   <211> 8
   <212> PRT
   <213> Artificial
<220>
   <223> chimeric antibody huHEA125, heavy chain, CDR1 domain
<400> 20
   Gly Phe Asp Phe Ser Arg Phe Trp
   1 5
<210> 21
   <211> 8
   <212> PRT
   <213> Artificial
<220>
   <223> chimeric antibody huHEA125, heavy chain, CDR2 domain
<400> 21
   Ile Asn Leu Asp Ser Ser Thr Ile
   1 5
<210> 22
   <211> 8
   <212> PRT
   <213> Artificial
<220>
   <223> chimeric antibody huHEA125, heavy chain, CDR3 domain
<400> 22
   Ser Arg Gly Ile Ser Met Asp Tyr
   1 5
<210> 23
   <211> 6
   <212> PRT
   <213> Artificial
<220>
   <223> chimeric antibody huHEA125, light chain, cDR1 domain
<400> 23
   Gln Ser Ile Gly Ile Ser
   1 5
<210> 24
   <211> 3
   <212> PRT
   <213> Artificial
<220>
   <223> chimeric antibody huHEA25, light chain, CDR2 domain
<400> 24
   Tyr Ala Ser
   1
<210> 25
   <211> 9
   <212> PRT
   <213> Artificial
<220>
   <223> chimeric antibody huHEA125, light chain, CDR3 domain
<400> 25
   Gln Gln Ser Asn Ile Trp Pro Thr Thr
   1               5
<210> 26
   <211> 399
   <212> PRT
   <213> Artificial
<220>
   <223> chimeric antibody huHEA125, heavy chain, constant domain, membrane-bound form
<400> 26
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Asp Glu
   225 230 235 240
   Leu Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Leu Gln Leu Asp Glu Thr Cys
   325 330 335
   Ala Glu Ala Gln Asp Gly Glu Leu Asp Gly Leu Trp Thr Thr Ile Thr
   340 345 350
   Ile Phe Ile Ser Leu Phe Leu Leu Ser Val Cys Tyr Ser Ala Ala Val
   355 360 365
   Thr Leu Phe Lys Val Lys Trp Ile Phe Ser Ser Val Val Glu Leu Lys
   370 375 380
   Gln Thr Leu Val Pro Glu Tyr Lys Asn Met Ile Gly Gln Ala Pro
   385 390 395
<210> 27
   <211> 330
   <212> PRT
   <213> Artificial
<220>
   <223> chimeric antibody huHEA125, heavy chain, constant domain, secreted form
<400> 27
<210> 28
   <211> 107
   <212> PRT
   <213> Artificial
<220>
   <223> chimeric antibody huHEA125, light chain, constant domain
<400> 28 1/15